(19) Europäisches Patentamt
European Patent Office
Office européen des brevets

(11) EP 1 712 637 A1

(12) **EUROPEAN PATENT APPLICATION**
published in accordance with Art. 158(3) EPC

(43) Date of publication:
**18.10.2006 Bulletin 2006/42**

(21) Application number: **04807253.2**

(22) Date of filing: **17.12.2004**

(51) Int Cl.:
*C12Q 1/37* (2006.01) *C12M 1/34* (2006.01)
*G01N 21/77* (2006.01) *G01N 21/78* (2006.01)

(86) International application number:
**PCT/JP2004/018895**

(87) International publication number:
**WO 2005/059166 (30.06.2005 Gazette 2005/26)**

(84) Designated Contracting States:
**AT BE BG CH CY CZ DE DK EE ES FI FR GB GR HU IE IS IT LI LT LU MC NL PL PT RO SE SI SK TR**

(30) Priority: **17.12.2003 JP 2003419346**

(71) Applicant: **Japan Science and Technology Agency**
**Kawaguchi-shi, Saitama 332-0012 (JP)**

(72) Inventors:
- **IWASAKI, Aki**
**Sunvarie Hiyoshi 9-605,**
**Yokohama-shi,**
**Kanagawa 223-0064 (JP)**
- **SUZUKI, Koji**
**Yokohama-shi Kanagawa 2200012 (JP)**

(74) Representative: **Gill, Stephen Charles et al**
**Mewburn Ellis LLP**
**York House**
**23 Kingsway**
**London WC2B 6HP (GB)**

# (54) METHOD AND APPARATUS FOR MEASURING ENVIRONMENTAL ALLERGEN

(57) A method for measuring an environmental allergen by which the allergen may be measured simply without using an anti-allergen antibody, as well as an instrument and apparatus therefor, is disclosed. In the method for measuring the environmental biological allergen, the biological allergen is measured by measuring the protease activity of the allergen. An instrument for measuring an environmental allergen(s) comprises a support and the substrate of the protease carried on the support, which substrate is used for the measurement of the protease activity of the allergen(s). The substrate is one which brings about fluorescence emission or change in absorption as a result of the enzyme reaction.

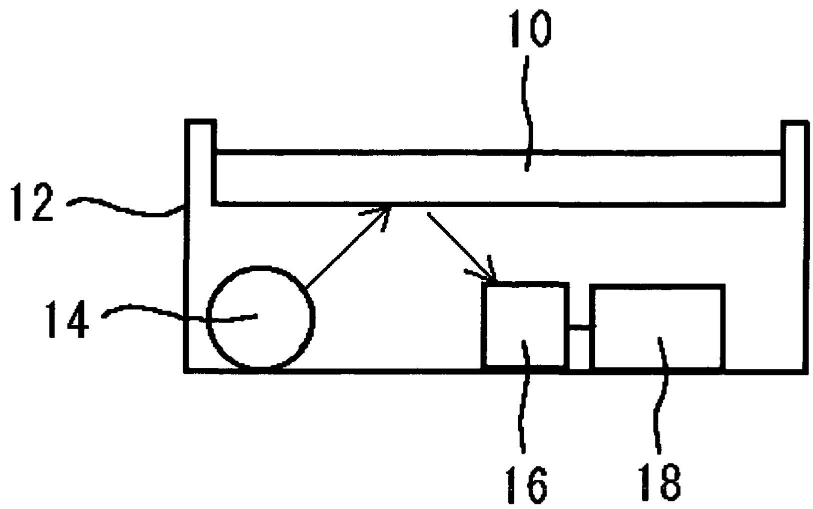


Fig.1

EP 1 712 637 A1

Printed by Jouve, 75001 PARIS (FR)

## Description

### Technical Field

The present invention relates to a method and apparatus for measuring environmental allergens.

### Background Art

It is well-known that pollen contained in atmosphere, mites contained in carpets and futon, and the like serve as allergens to cause allergic diseases such as pollinosis, atopy and asthma. It is important for those with allergic diathesis to avoid contact with these allergens in order to prevent onset of allergies.

Measurements of allergens have been conventionally carried out by immunoassays using the antibodies corresponding to the allergens to be measured (Patent Literatures 1 to 5 below).

However, antibodies, especially monoclonal antibodies used for increasing the measurement accuracy, are expensive.

On the other hand, it is known that there are environmental allergens originated from pollen, mites, molds, insects and the like, which have a protease activity. For example, it is known that cedar pollen (Non-patent Literature 1), ragweed pollen (Non-patent Literature 2), mesquite pollen (Non-patent Literature 3), *Aspergillus fumigatus* (Non-patent Literature 4) which is a kind of molds, allergen from *Periplaneta americana* (Non-patent Literature 5) and mites (*Dermatophagoides farinae* and *pteronyssinus*) (Non-patent Literatures 6 to 13) have a protease activity. However, these references do not disclose or suggest to measure the allergens utilizing the protease activity, and do not disclose or suggest that there is a quantitative relathionship between the protease activity and the amount of allergen. Further, in these references, the protease activities are measured after extracting, concentrating and/or purifying the allergens, and they do not disclose or suggest that environmental allergens may be simply measured as they are without these pretreatments.

Patent Literature 1: Japanese Laid-open Patent Application (Kokai) No. 9-87298
Patent Literature 2: Japanese Laid-open Patent Application (Kokai) No. 5-207892
Patent Literature 3: Japanese Laid-open Patent Application (Kokai) No. 11-14511
Patent Literature 4: Japanese Laid-open Patent Application (Kokai) No. 2000-35428
Patent Literature 5: Japanese Laid-open Patent Application (Kokai) No. 6-34518
Non-patent Literature 1: J Agric Food Chem. 2002 Jun 5;50(12):3540-3. Isolation and characterization of aminopeptidase (Jc-peptidase) from Japanese cedar pollen (Cryptomeriajaponica). Noguchi Y, Nagata H, Koganei H, Kodera Y, Hiroto M, Nishimura H, Inada Y, Matsushima A.
Non-patent Literature 2: Phytochemistry. 1998 Feb;47(4):593-8. Ragweed pollen proteolytic enzymes: possible roles in allergies and asthma. Bagarozzi DA Jr, Travis J.
Non-patent Literature 3: Am J Respir Cell Mol Biol. 1995 Apr;12(4):441-8. Isolation and properties of an angiotensin II-cleaving peptidase from mesquite pollen. Matheson N, Schmidt J, Travis J.
Non-patent Literature 4: J Investig Allergol Clin Immunol. 2002;12(4):257-62. Serine proteinases with gelatinolytic activity in an Aspergillus fumigatus allergenic extract. Iraneta SG, Duschak VG, Rodriguez SM, Alonso A.
Non-patent Literature 5: J Investig Allergol Clin Immunol. 1999 Jul-Aug;9(4):235-40. Proteinase and gelatinolytic activities of house dust mite and cockroach extracts. Iraneta SG, Duschak VG, Rodriguez SM, Seoane MA, Albonico JF, Alonso A.
Non-patent Literature 6: Ando T, Ino Y, Haida M, Honma R, Maeda H, Yamakawa H, Iwaki M, Okudaira H. Isolation of cysteine protease in the crude mite extract, Dermatophagoides farinae. Int Arch Allergy Appl Immunol. 1991;96 (3):199-205.
Non-patent Literature 7: Ando T, Homma R, Ino Y, Ito G, Miyahara A, Yamakawa H, Iwaki M, Okumura Y, Suko M, Haida M, et al. Is a trypsin-like protease of mites a Der f III allergen? Arerugi. 1992 Jun;41 (6):704-7.
Non-patent Literature 8: Ando T, Homma R, Ino Y, Ito G, Miyahara A, Yanagihara T, Kimura H, Ikeda S, Yamakawa H, Iwaki M, et al. Trypsin-like protease of mites: purification and characterization of trypsin-like protease from mite faecal extract Dermatophagoides farinae. Relationship between trypsin-like protease and Der f III. Clin Exp Allergy. 1993 Sep;23(9):777-84.
Non-patent Literature 9: King C, Simpson RJ, Moritz RL, Reed GE, Thompson PJ, Stewart GA. The isolation and characterization of a novel collagenolytic serine protease allergen (Der p 9) from the dust mite Dermatophagoides pteronyssinus. J Allergy Clin Immunol. 1996 Oct;98(4):739-47.
Non-patent Literature 10: Schulz O, Sewell HF, Shakib F. Related Articles, Links A sensitive fluorescent assay for measuring the cysteine protease activity of Der p 1, a major allergen from the dust mite Dermatophagoides pteronyssinus. Mol Pathol. 1998 Aug;51(4):222-4.

Non-patent Literature 11: Yasueda H, Mita H, Akiyama K, Shida T, Ando T, Sugiyama S, Yamakawa H. Allergens from Dermatophagoides mites with chymotryptic activity. Clin Exp Allergy. 1993 May;23(5):384-90.
Non-patent Literature 12: Heymann PW, Chapman MD, Aalberse RC, Fox JW, Platts-Mills TA. Antigenic and structural analysis of group II allergens (Der f II and Der p II) from house dust mites (Dermatophagoides spp). J Allergy Clin Immunol. 1989 Jun;83(6):1055-67.
Non-patent Literature 13: Stewart GA, Ward LD, Simpson RJ, Thompson PJ. Related Articles, Links The group III allergen from the house dust mite Dermatophagoides pteronyssinus is a trypsin-like enzyme. Immunology. 1992 Jan;75(1):29-35.

Disclosure of the Invention

Problems Which the Invention Tries to Solve

An object of the present invention is to provide a method for measuring allergens, by which environmental allergens may be measured simply without using an anti-allergen antibody, and to provide an instrument and apparatus therefor.

Means for Solving the Problems

The present inventors intensively studied to discover that environmental biological allergens may be simply measured by measuring the protease activity which the biological allergens such as mites and pollen have, even without a pretreatment such as extraction or condensation of the allergens, thereby completing the present invention.

That is, the present invention provides a method for measuring an environmental biological allergen(s), characterized by measuring said biological allergen(s) by measuring protease activity of said allergen(s). The present invention also provides an instrument for measuring a biological allergen(s), comprising a support and a substrate of a protease, which substrate is used for measuring protease activity of said allergen(s), and which substrate is carried on said porous support, said substrate being one which brings about fluorescence emission or change in absorption as a result of the enzyme reaction. The present invention further provides a measuring apparatus for measuring an environmental biological allergen(s), comprising a vessel containing a solution of substrate of protease, which substrate is used for the measurement of the protease activity of said allergen(s); and optical measuring device which measures fluorescence or the change in absorbance of said solution; said substrate being one which brings about fluorescence emission or change in absorption as a result of the enzyme reaction.

Effects of the Invention

By the present invention, a method for measuring allergens, by which environmental allergens may be measured simply without using an anti-allergen antibody, as well as an instrument and apparatus therefor, was first provided. Since the method of the present invention does not use an anti-allergen antibody, it may be carried out inexpensively. Further, since the method of the present invention may be carried out without a pretreatment of the allergen, the method is extremely simple and may be carried out without a skill. Still further, since the instrument and apparatus for measuring the biological allergens according to the present invention have a simple structure and are portable, measurement of allergens may be carried out *in situ* at the place such as home or school at which the allergens are desired to be measured. Therefore, it is expected that the present invention will greatly contribute to the prevention of allergic diseases such as atopy and pollinosis.

Brief Description of the Drawings

Fig. 1 schematically shows a preferred embodiment of the measuring apparatus according to the present invention.
Fig. 2 schematically shows another preferred embodiment of the measuring apparatus according to the present invention.
Fig. 3 schematically shows still another preferred embodiment of the measuring apparatus according to the present invention.
Fig. 4 schematically shows still another preferred embodiment of the measuring apparatus according to the present invention.
Fig. 5 schematically shows still another preferred embodiment of the measuring apparatus according to the present invention.

Fig. 6 schematically shows still another preferred embodiment of the measuring apparatus according to the present invention.

Fig. 7 schematically shows still another preferred embodiment of the measuring apparatus according to the present invention.

Fig. 8 shows the relationship between the concentration of mite bodies and fluorescence intensity, which was measured in an Example of the present invention.

Fig. 9 shows the relationship between the concentration of house dust and fluorescence intensity, which was measured in an Example of the present invention.

Fig. 10 is photographs showing the state resulted by attaching adhesive sheets to a carpet, window frame and floor corner, respectively, and then peeling off the sheets therefrom, as well as a control (no materials to be measured).

Fig. 11 is a photograph showing the fluorescent images immediately after transferring the adhesive sheets shown in Fig. 11 to the allergen-measuring sheet (gel) according to the present invention.

Fig. 12 is a photograph showing the fluorescent images immediately after transferring the adhesive sheets shown in Fig. 11 to the allergen-measuring sheet (gel) according to the present invention.

Fig. 13.1 is a phase contrast micrograph showing mite bodies to which a protease substrate was added, which micrograph was taken in an Example of the present invention.

Fig. 13.2 is a fluorescence micrograph showing mite bodies to which a protease substrate was added, which micrograph was taken in an Example of the present invention.

Fig. 14.1 is a phase contrast micrograph showing house dust to which a protease substrate was added, which micrograph was taken in an Example of the present invention.

Fig. 14.2 is a fluorescence micrograph showing house dust to which a protease substrate was added, which micrograph was taken in an Example of the present invention.

Fig. 15 shows the relationship between the concentration of the dust collected by a cleaner and absorbance, which was measured in an Example of the present invention.

Fig. 16 shows the relationships between the amounts of cedar pollen measured by using two types of substrates, respectively, and the fluorescence intensity, which relationships were measured in an Example of the present invention.

Fig. 17 shows the relationships between the amounts of cedar pollen extract, measured by using two types of substrates, respectively, and the fluorescence intensity, which relationships were measured in an Example of the present invention.

Fig. 18 shows the relationships between the amounts of mite extract, measured by using two types of substrates, respectively, and the fluorescence intensity, which relationships were measured in an Example of the present invention.

Fig. 19 shows spectra of the starting substance and reaction mixture before, after and during the enzyme reaction, respectively, the enzyme reaction being the reaction resulted by allowing aminopeptidase M to act on cresyl violet to which leucine molecule(s) was(were) bound through an amide bond(s).

Fig. 20 shows spectra of the starting substance and reaction mixture before and after the enzyme reaction, respectively, the enzyme reaction being the reaction resulted by allowing amide peptidase M to act on methylene violet to which leucine was bound through amide bond.

Fig. 21 shows spectra of the starting substance and reaction mixture before and after the enzyme reaction, respectively, the enzyme reaction being the reaction resulted by allowing aminopeptidase M to act on Safranin-O to which leucine molecule(s) was(were) bound through an amide bond(s).

Best Mode for Carrying Out the Invention

The measuring objects to be measured by the method of the present invention are environmental biological allergens. Here, "environment" means atmosphere and room air, as well as insides and outsides of floors, walls, windows, window frames, floor coverings (carpets, rugs, tatami mats, mats, straw mats and the like), beddings (futon, blankets, pillows, mattress and the like), fiber products such as fabrics, furnitures (chairs, sofas and the like), dust, house dust, river water, well water and the like, which may be a source of allergens to the room air, and foods and beverages are not included (drinking water is included). "Biological allergens" are living organisms *per se* and substances produced by living organisms, which cause allergies, such as pollens; bodies, feces, dead bodies and debris of mites and insects; molds and spores thereof; and the like.

In the method of the present invention, an allergen(s) is(are) measured by measuring the protease activity thereof. Therefore, the measuring object(s) of the method of the present invention is(are) an allergen(s) having protease activity. Preferred examples thereof include pollens, especially cedar pollen and mites (bodies, feces, dead bodies and debris). However, the allergens are not restricted thereto, and molds may also be the measuring objects.

In the method of the present invention, the protease activity of the above-described environmental biological

allergen(s) is measured. Although the fact *per se* that environmental biological allergens have protease activity is known, it was not known that there is a quantitative relationship between protease activity and the amount of an allergen, and that the allergen may be measured by measuring the protease activity. Further, surprisingly, as will be described concretely in Examples below, it was discovered by the present inventors that the protease activity of environmental biological allergens may be measured without any pretreatment such as extraction, concentration or purification of the allergens. Thus, in a preferred mode of the present invention, the environmental biological allergen(s) is(are) subjected to the measurement as it(they) is(are) or after merely being dissolved or suspended in water or in a buffer, without any pretreatment such as extraction or purification, so that the method is extremely simple. The term "measure" includes both quantification and detection.

The measurement of protease activity *per se* may be carried out by a known method. Various methods for measuring protease activity are known. Examples thereof include the method in which acid-denatured hemoglobin is used as a substrate; a method in which an acid soluble peptide generated by hydrolysis of casein is measured; a method in which a synthetic peptide, N-acetyl-L-phenylalanyl-3,5-diiodo-L-tyrosine (APDT) is used, and the liberated diiodo-L-tyrosine is quantified by ninhydrin method or the like; a method in which a peptide generated by an enzyme from a synthetic peptide is measured by high performance liquid chromatography; a method in which a synthetic peptide substrate, 4-methyl coumaryl-7-amide (MCA) is used; a method in which *p*-nitrophenyl ester synthetic peptide substrate or p-nitroanilide synthetic peptide substrate is used; a method in which ammonia or a carboxylic acid generated by hydrolysis is quantified; a method in which a substrate to which a pigment such as Congo red, azo, orcein is bound is used; a method in which radio activity is used; a method in which a peptide thioester is used; and gelatin film method. Among these methods, the methods employing a substrate of the enzyme, used for the measurement of the enzyme activity, which substrate is one that brings about fluorescence emission or change in absorption as a result of the enzyme reaction, are preferred because they are simple. In these cases, the protease activity may be measured by measuring the fluorescence or absorbance, or by visually observing the emission of fluorescence or the change in color.

The substrate which enables the measurement of protease activity based on the change in fluorescence characteristics of the substrate as a result of the enzyme reaction include compounds in which an oligopeptide(s) and/or amino acid(s) is(are) bound to a fluorescent substance. The fluorescence characteristics of these compounds change as a result of cleavage of the bond between the oligopeptide or amino acid and the other structure. A number of such substrates are commercially available. Such a substrate comprises a peptide fragment having a sequence composed by randomly combined one to about 10 appropriate amino acid residues, whose carboxyl terminal, amino terminal or intermediate site is bound through an amide bond to a substance (e.g., MCA, methylcoumaryl-7-amide) which emits fluorescence upon being cleaved and liberated; to two types of substances (e.g., combination of Dnp: 2,4-dinitrophenyl and MOCAc: 7-methoxycoumarin, and the like) with which fluorescence is quenched when the two substances exist in one molecule but fluorescence is emitted upon at least one of the substances is cleaved off; or to a substance (e.g., p-nitroanilide, benzoyl glycine, methyl ester and ethyl ester) which gives change in absorbance at a particular wavelength upon being liberated. The terminal of such substrates may be protected by succinyl group, acetyl group, *t*-butyroxycarbonyl group or the like. Examples of such substrates include, but not limited to, Arg-MCA, Boc-Ala-Gly-Pro-Arg-MCA (Boc represents t-butyroxycarbonyl), MOCAc-Arg-Pro-Lys-Pro-Tyr-Ala-Nva-Trp-Met-Lys(Dnp)-$NH_2$, Ac-Arg-OMe-HCl and Bz-Gly-Arg (Bz represents benzoyl) (All of these substrates are commercially available from PEPTIDE INSTITUTE INC). The substrates may be employed individually or in combination.

Since the substrate specificity of protease differs depending on the type of the allergen, the substrate with which the protease of the allergen to be measured reacts is selected and used. This selection may be carried out merely by a routine check test. In cases where mites (bodies, feces, dead bodies and/or debris) are measured, Boc-Val-Leu-Lys-MCA, Met-MCA, Boc-Gln-Ala-Arg-MCA, Boc-Val-Leu-Lys-MCA, Boc-Leu-Gly-Arg-MCA and the like, for example, may preferably be employed. In cases where pollen is measured, Bz-DL-Arg-pNA.HCl, Leu-MCA, Z-Arg-pNA, Z-Gly-Arg-pNA, Tyr-MCA and the like, for example, may preferably be employed. Further, exploiting the fact that substrate specificity differs depending on the type of the allergen, allergens may be separately measured. For example, as will be concretely described in an Example below, cedar pollen reacts with Leu-MCA or Bz-DL-Arg-pNA.HCl as a substrate, while does not react with Boc-Val-Leu-Lys-MCA. On the other hand, mite antigen reacts with Boc-Val-Leu-Lys-MCA as a substrate. Thus, cedar pollen and mite antigen may be distinguishingly measured using Leu-MCA and/or Bz-DL-Arg-pNA.HCl as the substrate(s) when measuring cedar pollen and using Boc-Val-Leu-Lys-MCA as the substrate when measuring the mite antigen. Further, protease inhibitors are also known. By making a protease inhibitor which inhibits the protease activity of a particular allergen coexist with the substrate used for the measurement, the protease activity of the particular allergen is eliminated, and the protease activity of the target allergen may be selectively measured. Examples of the protease inhibitors include p-methacrylbenzoic acid, diisopropyl fluorophosphate, tosylphenylalanyl chloromethyl ketone, subtilisin inhibitor, leupeptin, antipain, pepstatin and epoxy succinic acid derivatives. Thus, by appropriately selecting the substrate, and by making an inhibitor(s) against the protease of the allergen(s) which is desired to be excluded from the measurement, the type of the allergens to be measured may be considerably narrowed down.

The present inventors further invented pigments whose color is changed by the enzyme reaction by protease. That is, the present inventors discovered that when a protease acts on a colored compound which is a pigment having at least one amino group, in which an amino acid(s) and/or oligopeptide(s) is(are) bound to one or more of the at least one amino group through an amide bond(s), the amide bond(s) is(are) cleaved, that results in color change of the compound. The term "color change" herein means that both colors of the compound before and after the enzyme reaction can be visually seen, and the color change is discernible by visual observation. Color change may be more simply observed than fluorescence which requires excitation light, and even a slight change is more readily discernible than coloring (colorless compound is colored), so that observation of color change is advantageous. Preferred examples of the colored pigment include the pigments having an amino group(s) in a conjugated system, such as cresyl violet, Safranin O, methylene violet 3RAX, Nile blue A, Darrow red, Azure A, Azure C, Brilliant cresyl blue, rhodamine 123 and thionine. Especially preferred examples include, but not limited to, cresyl violet, Safranin O and methylene violet 3RAX, having the following chemical structures:

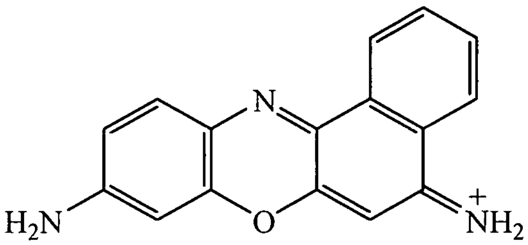

Cresyl Violet

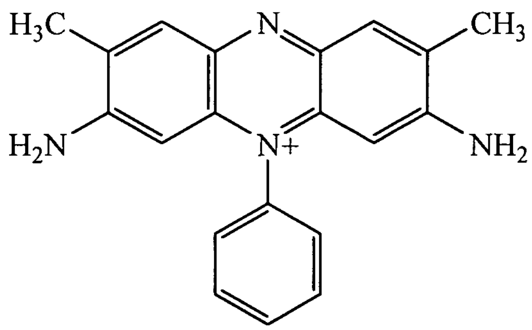

Safranin O

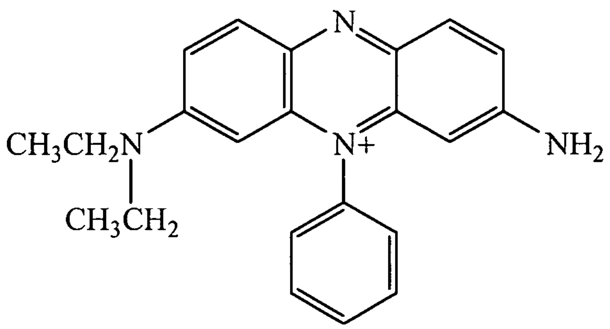

Methylene Violet 3RAX

In cases where the colored pigment has a plurality of amino groups as cresyl violet, it is sufficient if at least one of the amino groups is amidated. The amino acid to be subjected to amidation may be only one amino acid molecule or may be an oligopeptide (preferably one containing 2 to about 10 amino acids). The type of the amino acid and oligopeptide may appropriately be selected depending on the protease as described above. For example, for the measurement of *Dermatophagoides farinae* extract or *Dermatophagoides pteronyssinus* extract P, which is a mite antigen, those wherein one molecule of leucine, methionine or lysine is amidated are preferred, especially those wherein one or two amino groups of cresyl violet is bound to leucine by an amide bond(s) are preferred. It should be noted that the activity of cleaving the amide bond through which one molecule of amino acid is bound is endopeptidase activity, and it was first discovered by the present inventors that allergens such as mite antigen have endopeptidase activity.

The amide bond between the colored pigment and the amino acid may be attained by reacting the colored pigment molecule and the amino acid whose amino group is protected with Boc using as a condensation agent carbonyl diimidazole at room temperature for 1 day in methylene chloride or DMF, and after formation of the amide bond, deprotecting the amino group by trifluoroacetic acid. Detailed methods are described in Examples below. Oligopeptides may also be subjected to the formation of amide bond by the similar method.

The substrate, preferably the substrate which brings about fluorescence emission or change in absorption (including those whose color changes), may be used in the form of solution, or the substrate may be used in the form of being carried on a support. In case of solution, water or an aqueous buffer may preferably be used as the solvent. As the support, a porous support may preferably be used because a large amount of the substrate may be carried. Examples of the porous support include gels such as agarose gel, polyacrylamide gel and silica gel; filter papers made of glass fibers or the like; and porous films made of a synthetic resin. In cases where a substrate solution is used, although the concentration of the substrate in the solution may be appropriately selected depending on the type of the substrate used and the expected amount of the allergen, it is usually about 10 μM to 50 mM, preferably about 100 μM to 10 mM. Similarly, in cases where the substrate is carried on a support, although the amount of the substrate may appropriately be selected depending on the type of the substrate used and the expected amount of the allergen, it is usually about 1 nmol to 100 nmol per 1 $cm^2$ in cases where the substrate is applied to the surface of the support, and is usually about 0.1 μmol to 100 μmol in cases where the substrate is impregnated in the support. The cases where a substrate solution is used are suited for the measurement of allergen(s) in atmosphere or room air, and the allergen(s) in dust, house dust or the like which is(are) attached or contained in solids that may be suspended in the solution. However, since the dust collected by a cleaner may be suspended in the solution and the suspension may be subjected to measurement, by first cleaning floor covering or the like with an electric cleaner, and then subjecting the collected dust to the measurement after suspending the dust in the solution, the solution may be used for the measurement of the allergen(s) attached to, or contained in floors, walls, windows, window frames, floor coverings (carpets, rugs, tatami mats, mats, straw mats and the like), beddings (futon, blankets, pillows, mattress and the like), fiber products such as fabrics, and/or furnitures (chairs, sofas and the like). The cases where the substrate is carried on a support are suited for the measurement of allergen(s) attached to, or contained in floors, walls, windows, window frames, floor coverings, beddings, fiber products such as fabrics, and/or furnitures, in addition to the allergen (s) in atmosphere or room air. The support carrying thereon the substrate is convenient in carrying and storage, and measurement may be carried out simply, it is especially suited for the use in home, school or the like.

In cases where a substrate solution is used, the measurement may be attained by bringing atmosphere or room air into contact with the solution, or suspending the dust, house dust, the dust collected with an electric cleaner in the solution, and by measuring the fluorescnece or absorbance with a measuring apparatus or by visual observation. In cases where the substrate carried on a support is used, the measurement may be attained by bringing the support into direct contact with the floor, wall, window, window frame, floor covering, bedding, fiber product such as fabrics, and/or furniture, or bringing an adhesive sheet into contact with one or more of these and then bringing the adhesive sheet into contact with the support, and by measuring the fluorescnece or the color change with a measuring apparatus or by visual observation.

Preferred embodiments of the instrument and apparatus suited for the measuring method according to the present invention, as well as methods for using them will now be described, referring to the drawings as required.

The simplest measuring instrument is one comprising the support on which the substrate(s) is(are) carried. As the support, gels in the form of sheet, filter paper and the like are preferred. Such an allergen-measuring sheet is brought into direct contact with the floor, wall, window, window frame, floor covering, bedding, fiber product such as fabrics, and/or furniture, or an adhesive sheet is brought into contact with these and then the adhesive sheet is brought into contact with the support, and the fluorescence or the color change is visually observed. Alternatively, the allergen(s) in atmosphere or room air, freely falling on the sheet may also be measured. In cases where fluorescence is observed, the fluorescence is observed while illuminating the sheet with a lamp emitting the light having the excitation wavelength of the fluorescence.

Machine-measurement may be carried out when the allergen-measuring sheet is used. An embodiment of the measuring apparatus using the allergen-measuring sheet is schematically shown in Fig. 1. The upper opening of the

main body 12 of the apparatus, which is in the form of a hollow box is covered with an allergen-measuring sheet 10. The main body 12 of the apparatus contains a light source 14 which emits excitation light of the fluorescence. The lower surface of the sheet 10 is illuminated with the light source 14. The generated fluorescence is detected by a detector 16 contained in the main body 12 of the apparatus, and is digitized by a processor 18 connected to the detector 16. Here, the sheet 10 may be a porous sheet, a pump (not shown) may be contained in the main body 12 of the apparatus, and negative pressure may be applied to the inside of the main body 12 of the apparatus, thereby increasing the amount of the allergen(s) trapped by the sheet 10. This apparatus may be placed in a corner of a room or in atmosphere, and the allergen(s) freely falling on the allergen-measuring sheet may be measured. In this case, the allergen-measuring sheet may preferably be formed of a transparent material. Alternatively, the allergen-measuring sheet may be brought into direct contact with floor, wall, window, window frame, floor covering, bedding, fiber product such as fabrics, and/or furniture, or an adhesive sheet may be brought into contact with one or more of these and then the adhesive sheet may be brought into contact with the support. Then the upper opening of the measuring apparatus may be covered with the allergen-measuring sheet as shown in Fig. 1, and then machine-measurement may be carried out as described above. In this case, mounting the sheet such that the surface contacted with the allergen(s) faces downwardly is preferred because the fluorescence intensity is usually increased. With this apparatus, the allergen-measuring sheet may be provided with a frame, and, further, the frame may be provided with a grip, thereby the mounting of the sheet on the apparatus or the exchange of the sheet may be carried out easily.

Embodiments of the apparatus using the allergen-measuring sheet and further using a pump include those schematically shown in Figs. 2 and 3. With the apparatus shown in Fig. 2, an allergen-measuring sheet 34 is mounted in the main body 32 of the apparatus. The allergen-measuring sheet 34 may preferably be provided with a frame and, further, the frame may be provided with a grip, thereby making the sheet into the form of a cartridge. By constituting the apparatus such that the sheet made into the form of a cartridge is mounted on a cartridge holder, the allergen-measuring sheet may be exchanged easily, which is preferred. Above the sheet 34, a suction pipe 36 for introducing the sample air outside the main body 32 is arranged, and a pump 38 for drawing the outside air is connected to the suction pipe 36. On the outer open end of the suction pipe 36, a filter 40 is mounted and large dust is removed thereby. It is necessary that the size of the pores in the filter 40 be a size through which the allergen(s) can pass. Pump 38 is actuated to inhale the outside air into the suction pipe 36 so as to bring the allergen(s) contained in the air into contact with the allergen-measuring sheet 34. Thereafter, similar to the apparatus shown in Fig. 1, the measuring sheet 34 is illuminated with a light from a light source 42, the fluorescence or absorption is detected with a detector 44, and the results are digitized by a processor 46.

The measuring apparatus shown in Fig. 3 has a substantially the same constitution as the apparatus shown in Fig. 2, and the corresponding members are denoted by the same reference numerals as in Fig. 2. In the apparatus shown in Fig. 3, the light source 42 is arranged obliquely above the measuring sheet 34, and the light transmitting through the measuring sheet 34 is detected by the detector 44. In this case, the measuring sheet 34 is made of a transparent material.

Another embodiment is an allergen-measuring vessel in which a substrate solution is contained as it is, or in which the substrate solution absorbed in an absorbent made of a spongiform polymer or the like is contained. In this case too, as in the case of using the measuring sheet described above, the allergen(s) in atmosphere or room air, freely falling on the solution may be measured by visual observation.

An embodiment of an allergen-measuring apparatus utilizing such an allergen-measuring vessel is shown in Fig. 4. In the main body 20 of the apparatus, a transparent allergen-measuring vessel 22 containing a substrate solution 24 is contained. The solution 24 is illuminated with the light from a light source 26, the absorbance is measured with a detector 28, and the measurement results are digitized by a processor 30.

An embodiment of a measuring apparatus utilizing an allergen-measuring vessel and a pump is schematically shown in Fig. 5. In the main body 48 of the apparatus, an allergen-measuring vessel 50 containing a substrate solution 52 is mounted. The measuring vessel 50 is connected to a vessel 54 containing a buffer through a pipe 53. To the measuring vessel 50, a pump 56 is connected. A filter 58 is mounted in the buffer vessel 54 so as to prevent large dust from entering the measuring vessel 50. The buffer in the buffer vessel 54 may be stirred with a stirrer not shown. Above the buffer vessel 54, a suction pipe 60 is mounted, and the air outside the apparatus is introduced into the buffer vessel 54 by actuating the pump 62. The allergen(s) which entered the buffer in the buffer vessel 54 is introduced into the measuring vessel 50 through the pipe 53 by actuating the pump 56, and is(are) added to the substrate solution 52 together with the buffer. The substrate solution 52 is illuminated with the light from a light source 64, transmitted light or fluorescence is detected by a detector 66, and the results are digitized by a processor 68. Alternatively, in case of measuring fluorescence, the fluorescnece emitted in the direction perpendicular to the direction of the light from the light source 64 may be detected by a detector 70, and the results may be digitized with a processor 72. In this apparatus, the substrate solution may be contained in the measuring vessel 50, or the substrate in the form of powder may be attached to the bottom of the vessel. In the latter case, a substrate solution is prepared *in situ* with the buffer from the buffer vessel 54.

Another embodiment of a measuring apparatus using buffer is shown in Fig. 6. In the main body 74 of the apparatus, a vessel 78 in which a substrate 76 is placed on the bottom of the vessel 78 is mounted. The substrate 76 may be carried on a support to form an allergen-measuring sheet, and this sheet may be placed on the bottom of the vessel 78. Alternatively, the substrate in the form of powder may be merely attached to the bottom of the vessel 78. The vessel 78 is communicated to a buffer vessel 82 containing a buffer through a pipe 80. The buffer vessel 82 is equipped with a stirrer 84 and a filter 86. An openable and closable cover 90 is mounted on the upper side of the buffer vessel 82. A pump 88 is connected to the vessel 78. In operation, the cover 90 is opened, thereby leaving the buffer vessel 82 open for a prescribed time. Allergen(s) in the air enter(s) the buffer by free falling. After the prescribed time passed, the cover 90 is closed, and after stirring the buffer with the stirrer 84, the pump 88 is actuated to introduce the buffer into the vessel 78. By this, the buffer containing the allergen(s) contacts the substrate. The solution in the vessel 78 is illuminated with the light from a light source 90, the transmitted light or fluorescence is detected by a detector 92, and the results are measured by a processor 94. By this measuring apparatus, the measurement of, for example, "total amount of pollens in xx ski piste in the morning" or the like, may be attained accurately.

An embodiment of an apparatus using a buffer and a plurality of substrates, by which a plurality of allergens may be simultaneously measured, is schematically shown in Fig. 7. In the main body 96 of the apparatus, vessels 100a, 100b and 100c containing different types of substrates 98a, 98b and 98c, respectively, are mounted. The substrates 98a, 98b and 98c may preferably be different types of substrates by which different types of allergens may be measured. Each of the substrates may be carried on a support to form an allergen-measuring sheet, and the sheet may be placed on the bottom of each of the vessels 100a, 100b and 100c. Alternatively, the substrates in the form of powder may be attached to the bottom of the vessels 100a, 100b and 100c, respectively. The vessels 100a, 100b and 100c are communicated to a buffer vessel 104 containing a buffer through pipes 102a, 102b and 102c, respectively. A filter 106 is arranged in the buffer vessel 104. Above the buffer vessel 104, a suction pipe 110 is arranged, and the air outside the apparatus is introduced into the buffer vessel 104 by actuating the pump 108, so that the allergens in the air enter the buffer. The vessels 100a, 100b and 100c are connected to a pump 112. By actuating the pump 112, the buffer containing the allergens flows into the vessels 100a, 100b and 100c, thereby the allergens contact the each substrate. The vessels 100a, 100b and 100c are illuminated by light sources 114a, 114b and 114c, respectively, the fluorescence or absorbance is detected by detectors 116a, 116b and 116c, respectively, and the results are digitized by a processor 118. By this apparatus, a plurality of types of allergens may be simultaneously measured.

An apparatus comprising a main body in the form of a box, a pump placed in the main body, a filter covering the upper opening of the main body, and an air outlet formed in the main body may be used as an allergen-collecting apparatus. By actuating the pump to inhale the outside air into the inside of the main body through the filter, the allergen (s) in the air is(are) adsorbed to the filter. Then the allergen(s) is(are) liberated by immersing the filter in a substrate solution, or by washing the filter with a buffer, the washing solution is mixed with a substrate or substrate solution, and measurement is made. The measurement may be carried out by visual observation, by measuring absorbance using a commercially available spectrophotometer, or by measuring fluorescence using a fluorometer.

The present inventors will now be described more concretely by way of Examples thereof. It should be noted, however, the present invention is not limited to the Examples below.

Example 1

Quantification of Mite Antigen

(1) Materials
Frozen mite bodies (*Dermatophagoides farinae*) (produced by LSL)
Protease Substrate (Boc-Val-Leu-Lys-MCA) (produced by PEPTIDE INSTITUTE INC)
L-cysteine
(2) Methods and Results
Frozen mite bodies were suspended in phosphate buffer or in water to a concentration of 200 mg/ml, and the suspension was diluted with phosphate buffer as appropriate. To 180 μL of each suspension, 20 μL of 100 mM L-cysteine (generally added conventionally to reduce -SH groups of cystein protease to be measured) was added, and the resulting mixture was incubated at 37°C for 10 minutes. An aliquot of 40 μL of this mixture was mixed with 10 μL of the substrate dissolved in dimethyl sulfoxide (DMSO) to a concentration of 10 mM, and the resulting mixture was incubated at 37°C for another 30 minutes. To stop the reaction, 100 μL of 10% acetic acid solution was added. Using a plate reader, excitation was carried out at a wavelength of 355 nm, and fluorescence intensity at 460 nm was measured.
The relationship between the concentration of the mite bodies and the fluorescence intensity is shown in Fig. 8.

This curve is approximated to the following equation taking the number of mite bodies as x and taking the fluorescence intensity as y:

$$y=((A-D)/(1+(x/C)^B))+D$$

A=3.4402, B=1.456, C=70590.62, D=0.08607963

The correlation coefficient was 0.996. From this curve, it can be seen that mite antigen, at least within the range between about 2 mg/ml to 200 mg/ml, may be quantified by the method of this Example.

Example 2

Quantification of Mite Antigen in House Dust

(1) Materials

House Dust (dust collected with a cleaner)

Protease Substrate (Boc-Val-Leu-Lys-MCA) (produced by PEPTIDE INSTITUTE INC)

(2) Methods and Results

The dust collected with a cleaner was suspended in phosphate buffer to a concentration of 10 mg/ml, and the obtained suspension was filtered through a filter having a pore size of 1 μm. The obtained filtrate was 3-fold serially diluted 6 times. Each dilution in an amount of 40 μL was mixed with 2 μL of the substrate dissolved in DMSO to 10 mM, and the mixture was incubated at 37°C for 30 minutes. Using a plate reader, excitation was carried out at a wavelength of 355 nm, and fluorescence intensity at 460 nm was measured.

The relationship between the concentration (mg/ml) of the house dust and the fluorescence intensity is shown in Fig. 9. This curve is approximated to the following equation taking the concentration (mg/ml) of the house dust as x and taking the fluorescence intensity as y:

$$y=((A-D)/(1+(x/C)^B))+D$$

A=1203.294, B=1.374, C=6.672, D=44792.84

From this curve, it can be seen that mite antigen in house dust, at least within the range between about 1 mg/ml to 10 mg/ml, may be quantified by the method of this Example.

Example 3

Visualization of Mite Antigen on Floor or Carpet

(1) Materials

Protease Substrate (Boc-Val-Leu-Lys-MCA) (produced by PEPTIDE INSTITUTE INC)

L-cysteine

Agarose, Adhesive Sheet, Plastic Plate of 9 cm Diameter

Sample

Dust on Carpet, Vicinity of Window Frame or Floor Corner (all of them were collected in house room)

(2) Methods and Results

To 18 ml of phosphate buffer, 0.4 g of agarose was added and dissolved under heat with a microwave oven. The solution was cooled to 60°C and 2 ml of 100 mM L-cysteine was added. The solution was poured onto a plastic plate and cooled to solidify. To this agarose plate, 20 μL of the substrate dissolved in DMSO to a concentration of 10 mM was applied with a Conradi stick. Adhesive sheets with a size of several centimeters by several centimeters were attached to a carpet in a room, vicinity of window frame and floor corner, respectively, thereby collecting dust (Fig. 10). These sheets and an adhesive sheet as a control, which was not used for collecting dust, were attached to the above-described agarose plate, respectively. The agarose plate immediately after attaching the adhesive

sheets and after incubation for 30 minutes after attaching the adhesive sheets were excited with a light having a wavelength of 365 nm from a CCD camera, and the obtained image was observed with a UV illuminator. The results are shown in Figs. 11 and 12. In spite of the fact that infinite number of dust was observed visually in all cases, the detected dust was only a part thereof. It can be seen that the amount of the detected dust was large in the carpet and floor corner, and small in the vicinity of the window frame. Since the dust in the vicinity of the window frame is thought to include a large amount of sand or the like originated from the outside air, the results that the dust in the vicinity of the window frame contained only a small amount of mite antigen is agreeable. This Example is based on the quantification of cystein protease, and what was detected was mainly mite antigen.

Example 4

Observation of Mite with Fluorescence Microscope

(1) Materials
Protease Substrate (Boc-Val-Leu-Lys-MCA) (produced by PEPTIDE INSTITUTE INC)
L-cysteine
Sample
Frozen mite bodies *(Dermatophagoides farinae)* (produced by LSL)
(2) Methods and Results

A small amount of frozen mite bodies or dust collected with a cleaner was sampled on a Petri dish for observation, and 36 μL of phosphate buffer, 4 μL of L-cysteine and 5 μL of the substrate were added, followed by observation with microscopes to obtain a phase contrast image or fluorescent image (excitation: Ar laser, CFP filter). The results are shown in Figs. 13.1 and 13.2. From comparison of phase contrast image (Fig. 13.1) and fluorescent image (Fig. 13.2), it can be seen that the fluorescence intensity was especially high in the digestive organs in the mite bodies. This suggests that the allergens including Derf1 were specifically detected.

Example 5

Visualization of Mite Antigen in House Dust

(1) Materials
Protease Substrate (Boc-Val-Leu-Lys-MCA) (produced by PEPTIDE INSTITUTE INC)
L-cysteine
Sample
Dust Collected with Air Cleaner
(2) Methods and Results

Dust collected with a cleaner was sampled on a Petri dish for observation, and 36 μL of phosphate buffer, 4 μL of L-cysteine and 5 μL of the substrate were added, followed by observation with microscopes to obtain a phase contrast image or fluorescent image (excitation: Ar laser, CFP filter). The results are shown in Figs. 14.1 and 14.2. From comparison of phase contrast image (Fig. 14.1) and fluorescent image (Fig. 14.2), it can be seen that fluorescence was detected not in all of the dust collected with the cleaner, but in only a part thereof. It is thought that mainly mite antigen was detected.

Example 6

Quantification of Mite Antigen in House Dust by Visual Observation or Measurement of Absorbance

(1) Materials
House Dust (dust collected with a cleaner)
Protease Substrate (Bz-DL-Arg-pNA, HCl) (produced by PEPTIDE INSTITUTE INC)

(2) Methods and Results

The dust collected with a cleaner was suspended in phosphate buffer to a concentration of 10 mg/ml and the obtained suspension was filtered through a filter having a pore size of 1 μm. The obtained filtrate was 3-fold serially diluted 6 times. Each dilution in an amount of 180 μL was mixed with 20 μL of the substrate dissolved in DMSO to a concentration of 10 mM, and the mixture was incubated at 37°C overnight. The colored plate was scanned from the downside with a scanner. As a result, coloring in yellow was observed in the wells containing a sample with a high concentration. The absorbance at 405 nm was measured with a plate reader. The results are shown in Fig. 15. This curve is approximated to the following equation taking the amount of the dust collected with the cleaner as x and taking the absorbance as y:

$$y=((A-D)/(1+(x/C)^B))+D$$

A=0.061, B=1.398, C=5.059, D=0.575
From this curve, it can be seen that mite antigen in house dust, at least within the range between about 1 mg/ml to 10 mg/ml, may be quantified by the method of this Example.

Example 7

Measurement of Cedar Pollen

(1) Materials
Cedar Pollen (Cedar Pollen-Cj Japanese Cedar) (produced by LSL)
Leu-MCA and Boc-Val-Leu-Lys-MCA as protease substrates (both of them were produced by PEPTIDE INSTITUTE INC)
(2) Methods and Results

Cedar pollen was suspended in phosphate buffer to a concentration of 1 mg/ml and the suspension was diluted with phosphate buffer as appropriate. Each of the dilutions in an amount of 45 μL was mixed with 5 μL of the substrate dissolved in DMSO to a concentration of 10mM, and the resulting mixture was incubated at 37°C for 15 minutes. Using a plate reader, excitation was carried out at a wavelength of 355 nm, and fluorescence intensity at 460 nm was measured.
The results are shown in Fig. 16. The curve obtained when Leu-MCA was used is approximated to the following equation taking the amount (μg/ml) of the cedar pollen as x and taking the fluorescence intensity as y:

$$y=((A-D)/(1+(x/C)^B))+D$$

A=3943.221, B=1.374, C=169.224, D=4939.37
The correlation coefficient was 0.997.
From this curve, it can be seen that cedar pollen, at least within the range between about 50 μg/ml to 1000 μg/ml, may be quantified by the method of this Example.
Further, as is also apparent from Fig. 16, when Boc-Val-Leu-Lys-MCA was used, the fluorescence was not dependent on the amount of cedar pollen. From this, it can be seen that the substrate specificity differs depending on the type of the biological allergens, and biological allergens may be specifically detected by using different substrates for different allergens.

Example 8

Quantification of Cedar Pollen Extract

(1) Materials

Cedar Pollen Extract (Cedar Pollen Extrace-Cj) (produced by LSL)
Leu-MCA and Boc-Val-Leu-Lys-MCA as protease substrates (both of them were produced by PEPTIDE INSTITUTE INC)
(2) Methods and Results

The cedar pollen extract was the centrifugation supernatant obtained by stirring cedar pollen in 0.125 M $NaHCO_3$ (pH8) for 16 hours, and lightly homogenizing the mixture, followed by centrifugation. The extract was suspended in 5 mM borate buffer (pH8.0) containing 0.9% NaCl to a concentration of 666 μg/ml, and the resulting mixture was diluted with phosphate buffer as appropriate. Each of the samples in an amount of 45 μL was mixed with 5 μL of the substrate dissolved in DMSO to a concentration of 10mM, and the resulting mixture was incubated at 37°C for 15 minutes. Using a plate reader, excitation was carried out at a wavelength of 355 nm, and fluorescence intensity at 460 nm was measured.
The results are shown in Fig. 17. The curve obtained when Leu-MCA was used is approximated to the following equation taking the amount (μg/ml) of the cedar pollen as x and taking the fluorescence intensity as y:

$$y=((A-D)/(1+(x/C)^B))+D$$

A=3134.98, B=0.932, C=371.278, D=25315.2
The correlation coefficient was 1, and it was shown that the detection limit was improved by using the extract when compared with the case wherein the pollen was used as it is. Similar to Example 7, when Boc-Val-Leu-Lys-MCA was used, the fluorescence was not dependent on the amount of cedar pollen. This indicates that detection of the pollen may be attained with specificity even if the pollen is physically disrupted.

Example 9

(1) Materials
Mite Extract Df (Mite dermatophagoides farinae) (obtained by extracting mites with phosphate buffer and freeze-drying the soluble fragment) (produced by LSL) Protease Substrates (Met-MCA, Boc-Gln-Ala-Arg-MCA, Boc-Val-Leu-Lys-MCA, Bz-Arg-MCA, Glt-Ala-Ala-Phe-MCA (Glt means glutaryl) (All of them were produced by PEPTIDE INSTITUTE INC)
(2) Methods and Results

In phosphate buffer or water, mites were suspended to a concentration of 1 mg/ml, and the suspension was diluted with phosphate buffer as appropriate. Each of the dilutions in an amount of 50 μL was mixed with 5 μL of the substrate dissolved in DMSO to a concentration of 10mM, and the resulting mixture was incubated at room temperature overnight. Using a plate reader, excitation was carried out at a wavelength of 355 nm, and fluorescence intensity at 460 nm was measured.
The results are shown in Fig. 18. These curves show that mite antigen may be effectively measured by using a substrate such as Met-MCA, Boc-Gln-Ala-Arg-MCA or Boc-Val-Leu-Lys-MCA by the method of this Example. Further, a substrate such as Bz-Arg-MCA or Glt-Ala-Ala-Phe-MCA was not substantially cleaved by mite antigen, so that it was shown that the substrate was not cleaved non-specifically.

Example 10

Quantification of Mite Antigen

Substrates which change their color by cleavage by an enzyme were synthesized. To each of the amino groups of cresyl violet, Safranin O and methylene violet 3RAX, leucine or methionine was bound through an amide bond(s). That is, the pigment molecules (final concentration: 0.1M), the amino acid (final concentration: 0.2M) whose amino group was protected with Boc, and carbonyl diimidazole (final concentration 0.1 M) as a condensing agent, were reacted at room temperature for one day in methylene chloride or DMF, thereby forming an amide bond(s). After the reaction, the amino group(s) was(were) deprotected using trifluoroacetic acid (50%)/methylene chloride (50%).
Cresyl violet has two amino groups. By the above-described synthesis process, the compound (yellow, maximum absorption wavelength: 440 nm) in which both of the amino groups were bound to the amino acid through amide bonds, and the compound (orange, 490 nm) in which only one of the amino groups was bound to the amino acid through

an amide bond, were isolated. The absorption wavelength of each compound was measured with a plate reader (SPECTRA Max).

An experiment to cleave the amino bond(s) in the compounds between leucine and cresyl violet was carried out by adding aminopeptidase M. That is, to 50 μL of a solution of aminopeptidase M, an enzyme which cleaves off the N-terminal amino acid bound through amide bond, diluted with PBS as appropriate, 5 μL of the substrate dissolved in ethanol to a concentration of about 10 mM was added. After leaving the mixture to stand for 30 minutes, absorption spectrum was measured using a 96-well plate. As a result, the absorption spectrum of the substrate in which only one of the amino groups contributed to the formation of amide bond changed from orange (maximum absorption wavelength: 490 nm) to violet (maximum absorption wavelength: 590 nm) upon digestion with the enzyme, and the absorption spectrum of the substrate in which both amino groups contributed to the formation of amide bond changed from yellow (maximum absorption wavelength: 450 nm) to violet (maximum absorption wavelength: 590 nm) via blue. Mass spectrometry revealed that the blue substance was the compound in which one leucine molecule was bound to cresyl violet. The violet absorption spectrum finally attained by the enzyme digestion was completely coincide with that of cresyl violet *per se,* and it was also confirmed by thin layer chromatography that the substance was cresyl violet itself. The reason why the compound in which cresyl violet is bound with one leucine molecule gives two different colors of blue and orange is that the molecular structure of cresyl violet is not symmetric, and the absorption spectrum differs depending on which amino group participates in the formation of the amide bond. The respective absorption spectra are shown in Fig. 19. It was confirmed that similar color change occurred when methionine or lysine was used in place of leucine.

Methylene violet 3RAX has one amino group. When the amino group contributes to the formation of amide bond with an amino acid, its color was light violet (maximum absorption wavelengths: 550 nm and 590 nm), and upon digestion with aminopeptidase M, its color changed to bright pink (maximum absorption wavelength: 550 nm). The absorption spectrum after digestion was completely coincide with that of methylene violet 3RAX. The manner of spectrum change of the binding product of methylene violet 3RAX with leucine through amide bond, upon digestion with aminopeptidase M, is shown in Fig. 20. In Fig. 20 and in Fig. 21 described below, the term "Apase" means aminopeptidase M.

The binding product of Safranin O with leucine through amide bonds has two amino groups, and binding product of this compound with leucine showed red color. Upon digestion with aminopeptidase M, it colored in orange. The absorption spectrum after the digestion was completely coincide with that of Safranin O. The manner of spectrum change of the binding product of Safranin O with leucine through amide bonds, upon digestion with aminopeptidase M, is shown in Fig. 21.

It was confirmed that the binding product (CV-Leu) of cresyl violet with leucine through amide bond changed its color depending on the amount of mite antigen in house dust. That is, the samples a to g shown in Table 1 were checked for the amount of mite antigen therein using a commercially available ELISA kit for measuring indoor allergens in accordance with the package insert. To the samples a to g, CV-Leu was added and the resulting mixtures were left to stand overnight. As a result, the samples a and b colored in violet and were shown to contain a large amount of mite antigen. These results agreed with the results of ELISA. Thus, it was proved that mite antigen can be measured using CV-Leu as a substrate based on the color change thereof.

Table 1

| | Derf1 | Derp1 | Der2 | Total |
|---|---|---|---|---|
| a | 2367 | 20.6 | 60 | 2447.6 |
| b | 8367 | 171.8 | 202.8 | 8741.6 |
| c | 1567 | 18.4 | 53.8 | 1639.2 |
| d | 1831 | - | 18.6 | 1849.6 |
| e | 15.31 | - | - | 15.31 |
| f | 1199 | 17.9 | 32.2 | 1249.1 |
| g | 725 | 9.7 | 20.4 | 755.1 |

Industrial Availability

The method, measuring instrument and measuring apparatus according to the present invention make it possible to simply measure environmental allergens such as mites and pollens, and are useful for the prevention of various allergies and the like.

## Claims

1. A method for measuring an environmental biological allergen(s), **characterized by** measuring said biological allergen (s) by measuring protease activity of said allergen(s).

2. The method according to claim 1 or 2, wherein said allergen(s) is(are) mite and/or a material(s) originated from mite, and/or pollen.

3. The method according to claim 2, wherein said pollen is cedar pollen.

4. The method according to any one of claims 1 to 3, by which an allergen(s) in atmosphere, room air, floor(s), wall (s), window(s), window frame(s), floor covering(s), bedding(s), fiber product(s), furnitures, dust and/or house dust is(are) measured.

5. The method according to any one of claims 1 to 4, wherein substrate of the enzyme, used for the measurement of the enzyme activity is a substrate which brings about fluorescence emission or change in absorption as a result of the enzyme reaction.

6. The method according to any one of claims 1 to 5, wherein said allergen(s) in a test material(s) is(are) brought into contact with the substrate of said enzyme without a pretreatment.

7. The method according to claim 6, wherein said substrate is carried on a support, and the test material(s) is(are) brought into contact with said support.

8. The method according to claim 6, wherein said support is a porous support.

9. An instrument for measuring a biological allergen(s), comprising a porous support and a substrate of a protease, which substrate is used for measuring protease activity of said allergen(s), and which substrate is carried on said porous support, said substrate being one which brings about fluorescence emission or change in absorption as a result of the enzyme reaction.

10. The instrument according to claim 9, wherein said allergen(s) is(are) mite and/or a material(s) originated from mite, and/or pollen.

11. A measuring apparatus for measuring an environmental biological allergen(s), comprising said instrument according to claim 9 and 10, and an optical measuring device which measures fluorescence or the change in absorbance of said porous support.

12. The measuring apparatus according to claim 11, further comprising a guide which guides a test material(s) to said instrument.

13. A measuring apparatus for measuring an environmental biological allergen(s), comprising a vessel containing solution of substrate of protease, which substrate is used for the measurement of the protease activity of said allergen (s); and optical measuring device which measures fluorescence or the change in absorbance of said solution; said substrate being one which brings about fluorescence emission or change in absorption as a result of the enzyme reaction.

14. The measuring apparatus according to claim 13, further comprising a guide which guides a test material(s) to said instrument.

15. The measuring apparatus according to claim 14, wherein said allergen(s) to be measured is(are) guided to a buffer contained in a buffer vessel; and said buffer is guided to said solution.

16. A measuring apparatus for measuring an environmental biological allergen(s), comprising a vessel containing substrate of protease, which substrate is used for measuring protease activity of said allergen(s); and an optical measuring device which measures fluorescence or the change in absorbance of the solution; said substrate being one which brings about fluorescence emission or change in absorption as a result of the enzyme reaction.

**17.** The measuring apparatus according to claim 16, wherein said allergen(s) to be measured is(are) guided to a buffer contained in a buffer vessel; and said buffer is guided to said solution.

**18.** The method according to claim 5, wherein said substrate is a colored compound which is a pigment having at least one amino group, an amino acid(s) and/or oligopeptide(s) being bound to one or more of said at least one amino group through an amide bond(s).

**19.** The method according to claim 18, wherein said substrate is a colored compound which is a pigment having at least one amino group, an amino acid(s) being bound to one or more of said at least one amino group through an amide bond(s).

**20.** The method according to claim 18 or 19, wherein said pigment is cresyl violet, Safranin O or methylene violet 3RAX.

**21.** The instrument according to claim 9, wherein said substrate is a colored compound which is a pigment having at least one amino group, an amino acid(s) and/or an oligopeptide(s) being bound to one or more of said at least one amino group through an amide bond(s).

**22.** The instrument according to claim 21, wherein said substrate is a colored compound which is a pigment having at least one amino group, an amino acid(s) being bound to one or more of said at least one amino group through an amide bond(s).

**23.** The instrument according to claim 21 or 22, wherein said pigment is cresyl violet, Safranin O or methylene violet 3RAX.

**24.** The measuring apparatus according to any one of claims 9 to 17, wherein said substrate is a colored compound which is a pigment having at least one amino group, an amino acid(s) and/or an oligopeptide(s) being bound to one or more of said at least one amino group through an amide bond(s).

**25.** The measuring apparatus according to claim 24, wherein said substrate is a colored compound which is a pigment having at least one amino group, an amino acid(s) being bound to one or more of said at least one amino group through an amide bond(s).

**26.** The measuring apparatus according to claim 18 or 19, wherein said pigment is cresyl violet, Safranin O or methylene violet 3RAX.

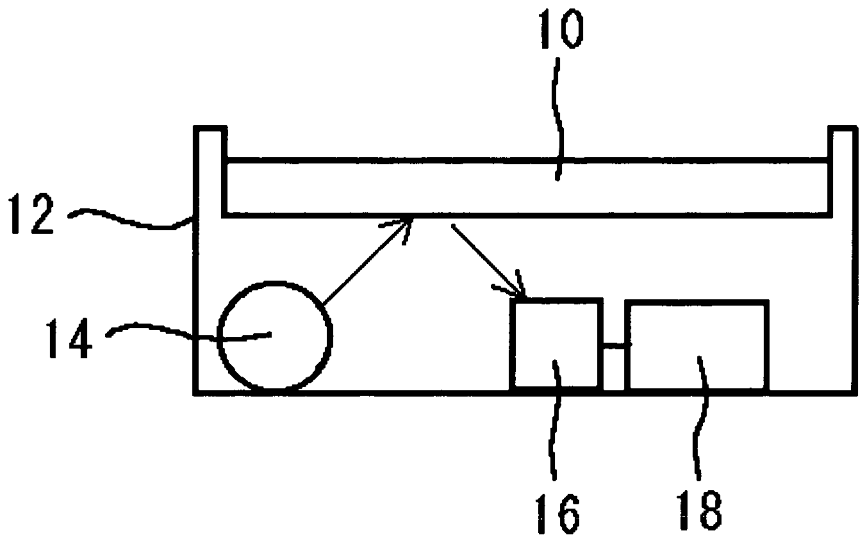


Fig.1

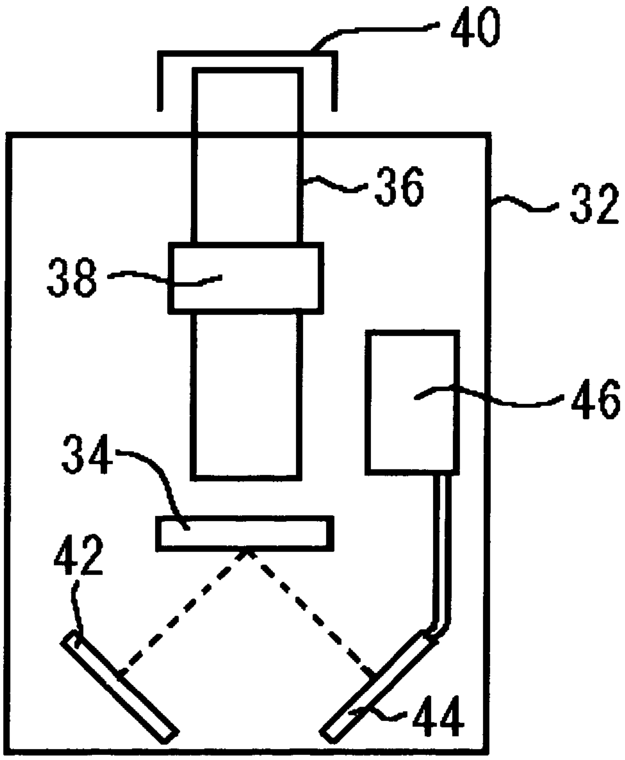


Fig.2

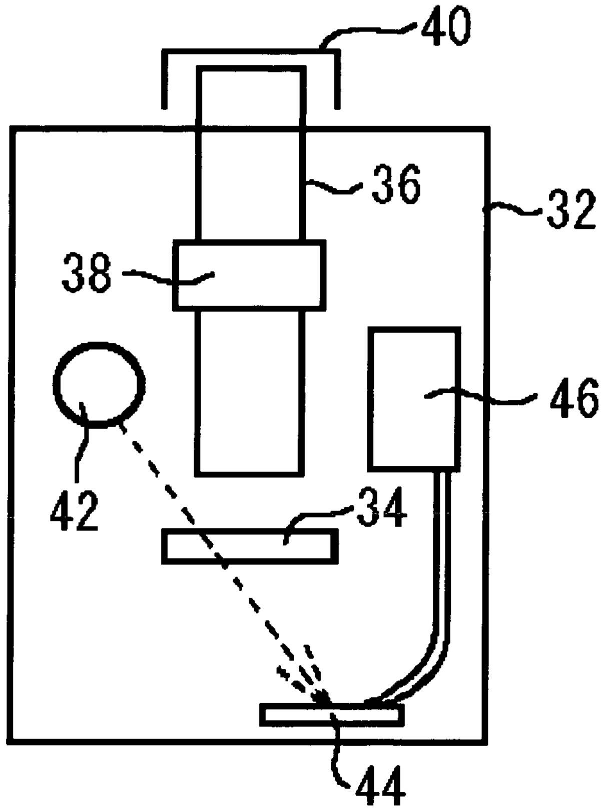


Fig.3

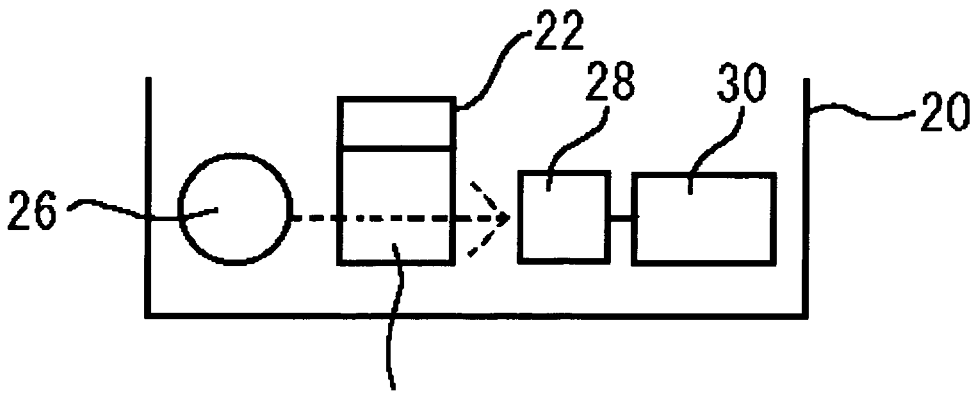


Fig.4

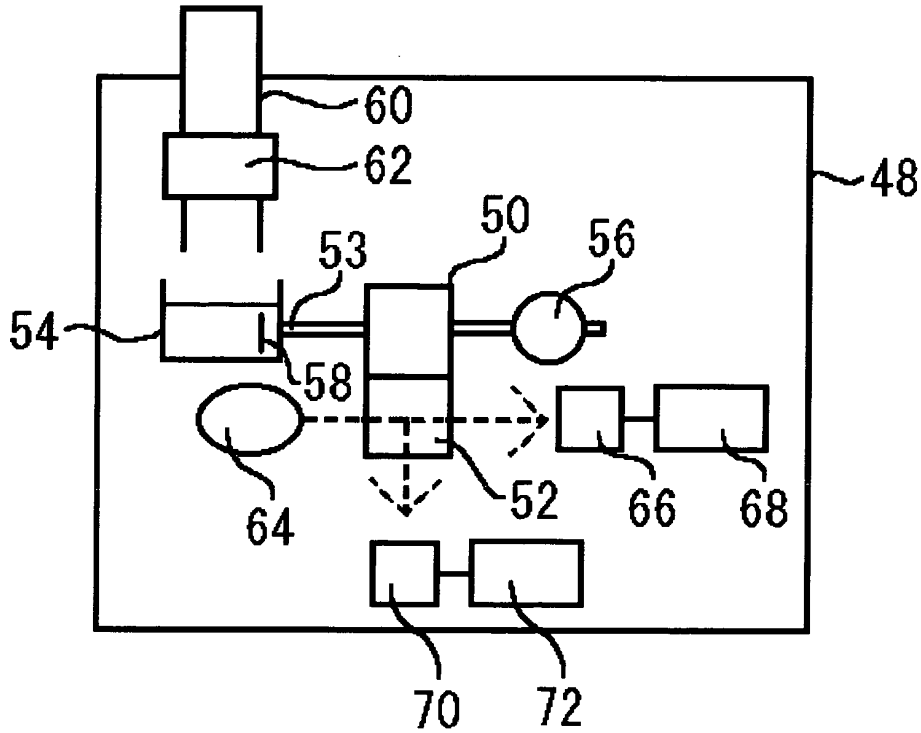
60
62
48
50
56
53
54
58
64
52
66
68
70
72

Fig.5

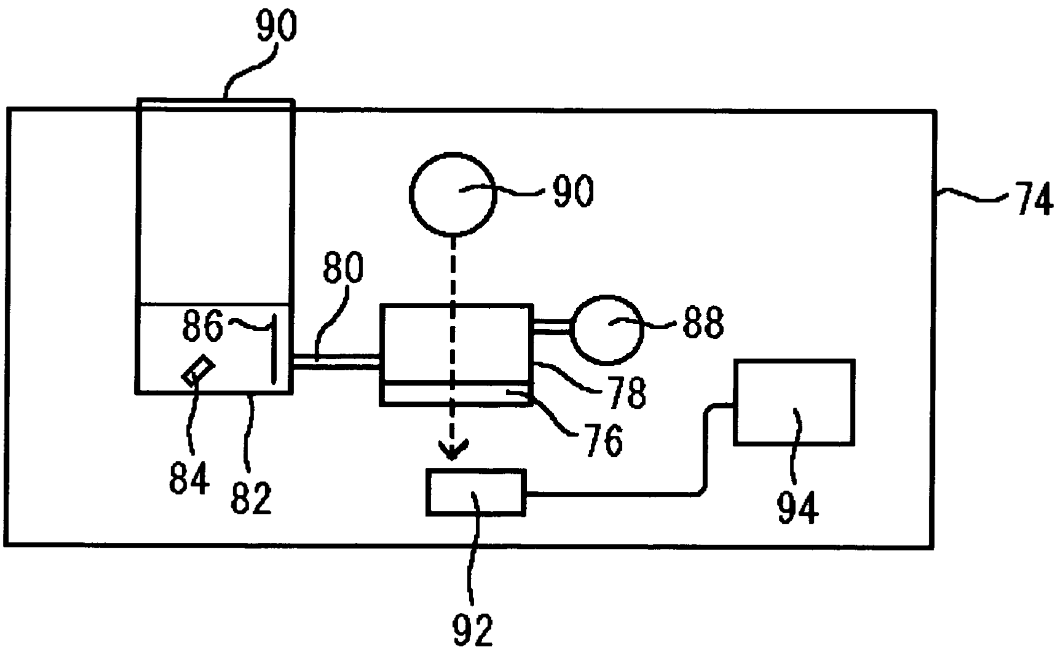
90
90
74
80
86
88
78
76
84
82
94
92

Fig.6

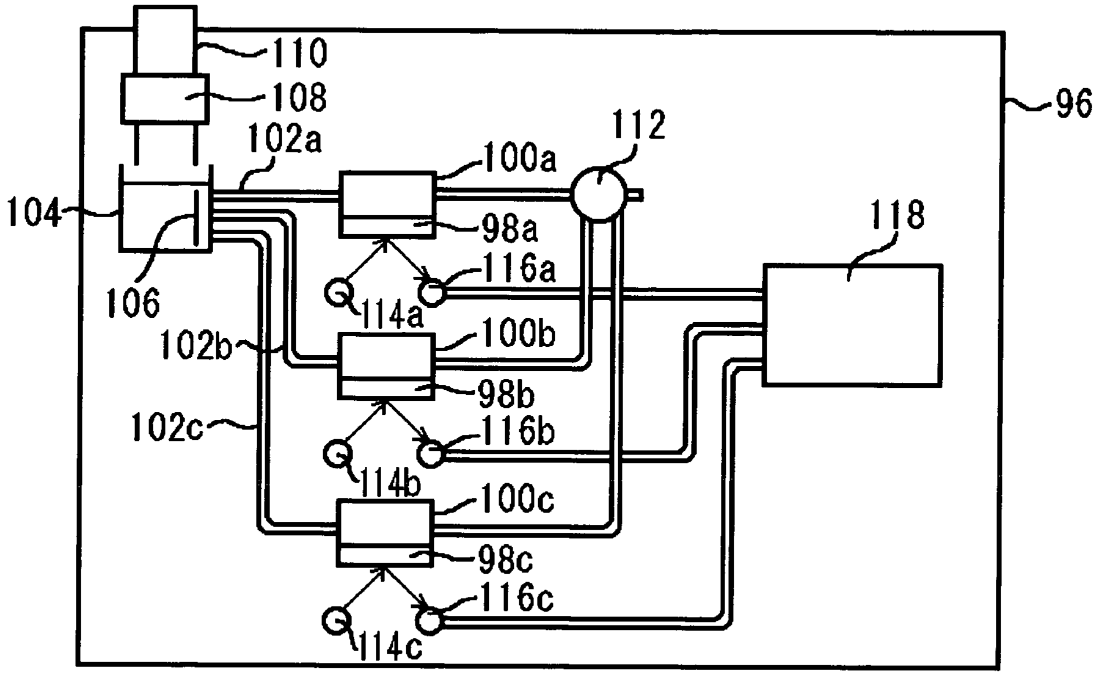
110
108
102a
112
96
104
100a
98a
118
106
116a
114a
102b
100b
98b
102c
116b
114b
100c
98c
116c
114c

**Fig.7**

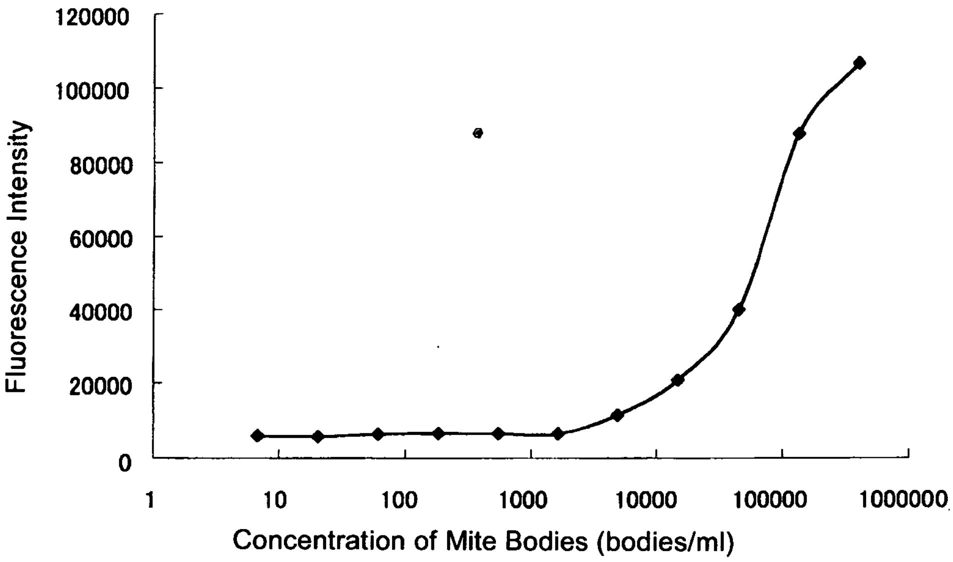
120000
100000
80000
60000
40000
20000
0
Fluorescence Intensity
1
10
100
1000
10000
100000
1000000
Concentration of Mite Bodies (bodies/ml)

**Fig.8**

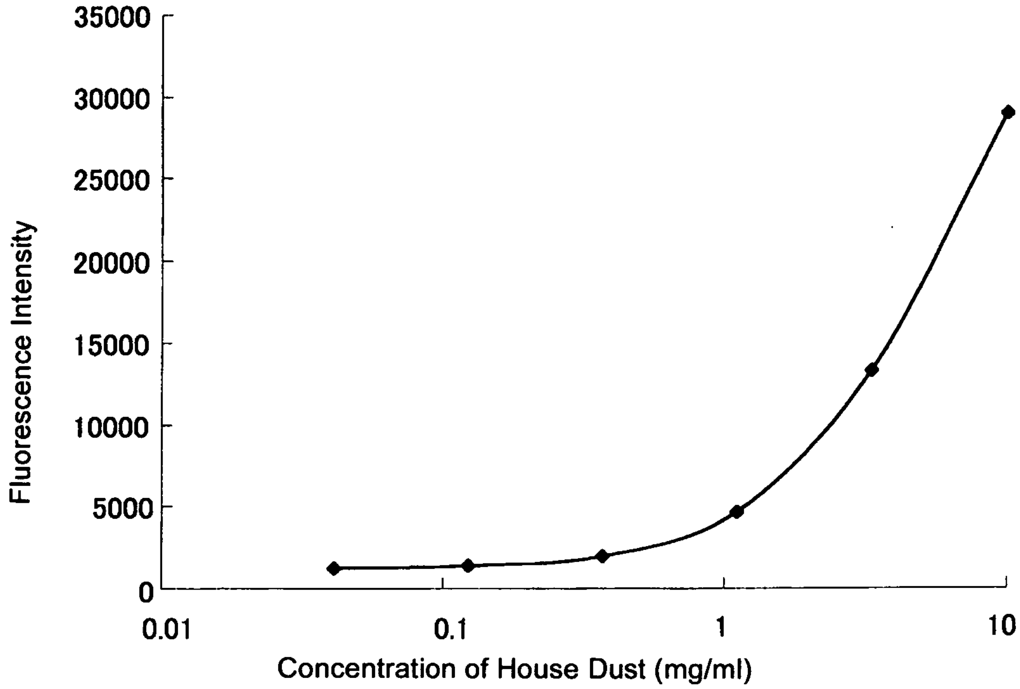
35000
30000
25000
20000
15000
10000
5000
0
Fluorescence Intensity
0.01
0.1
1
10
Concentration of House Dust (mg/ml)

**Fig.9**

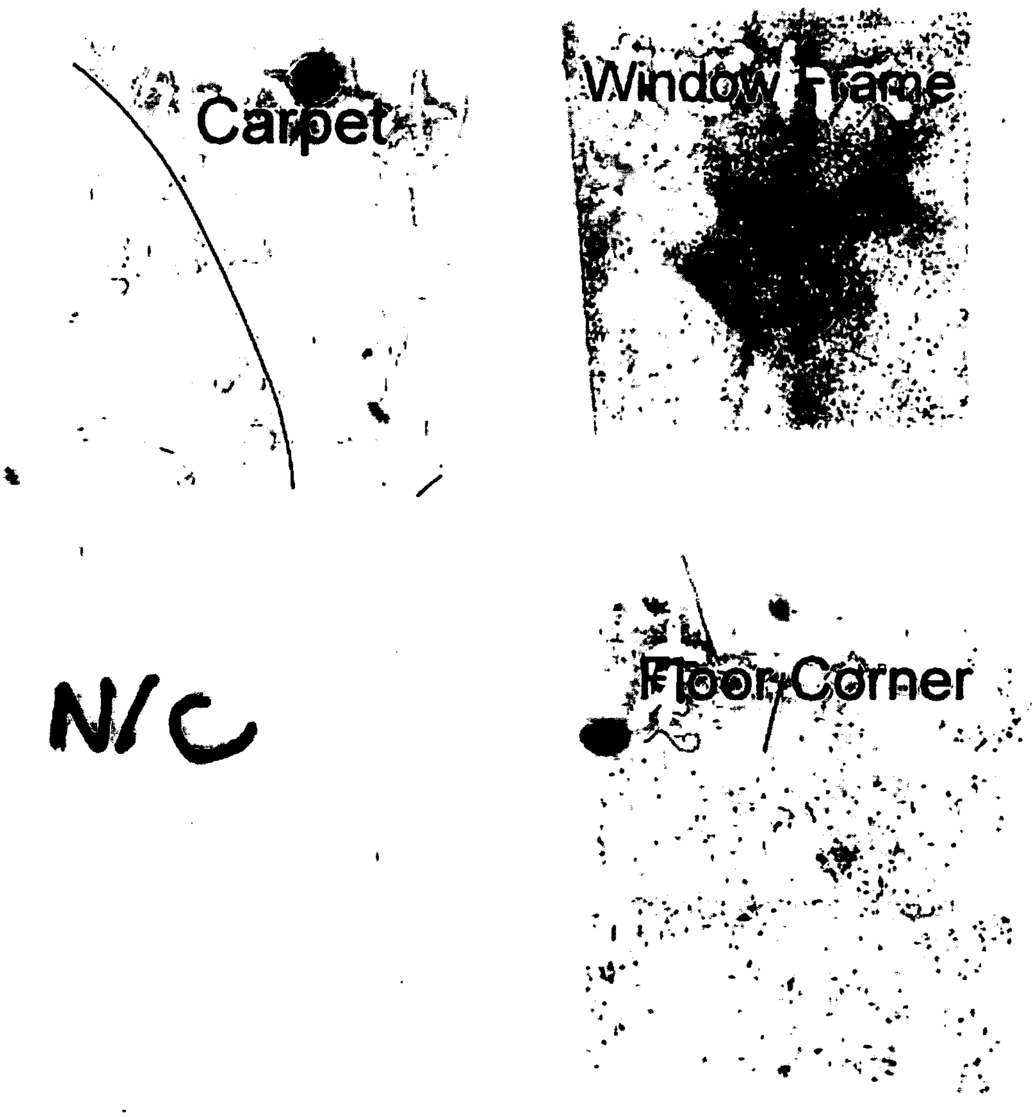


Fig.10

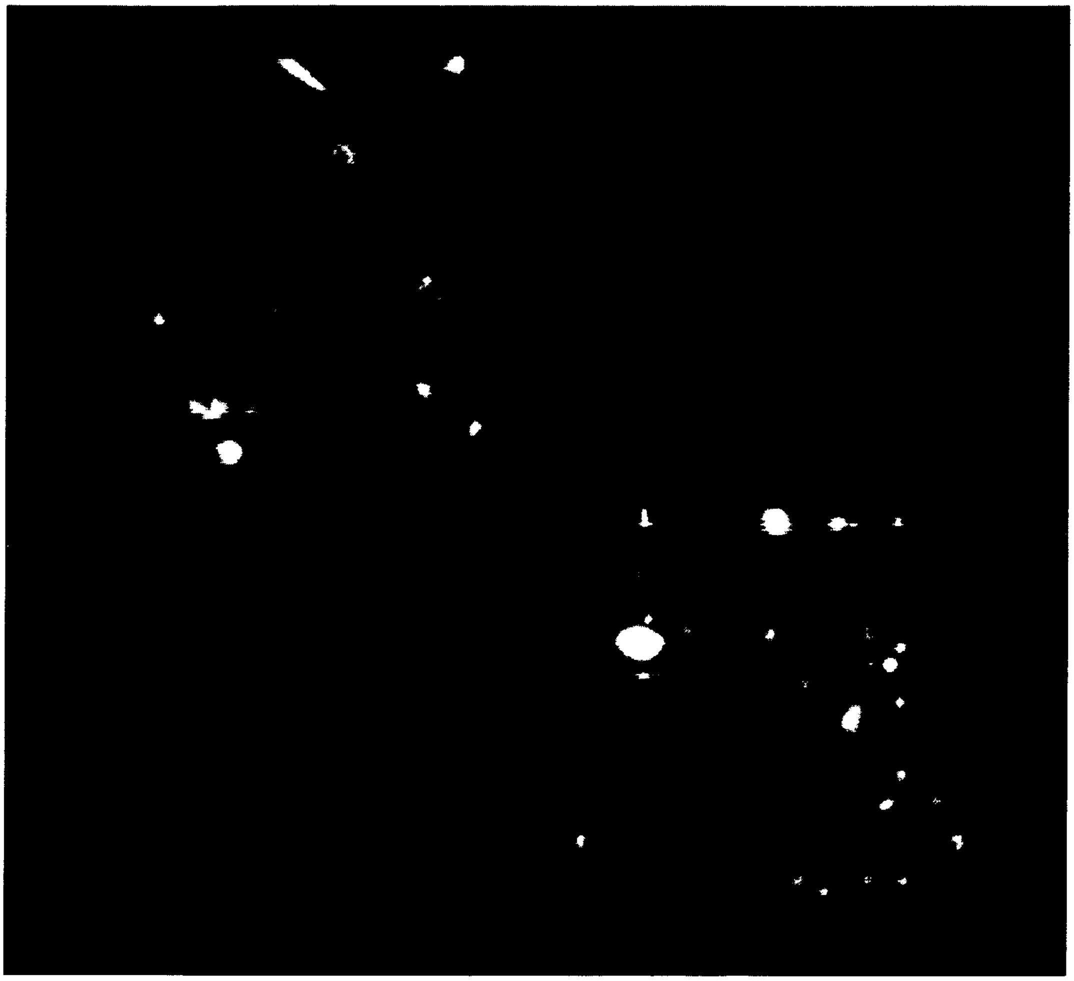

Fig.11

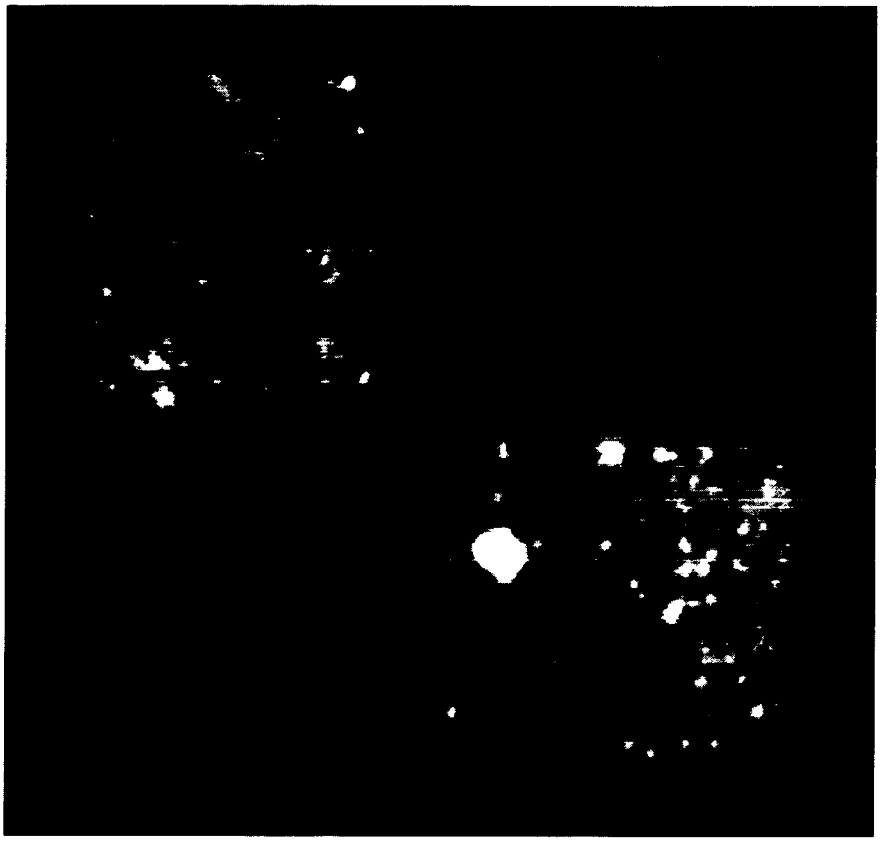

Fig.12

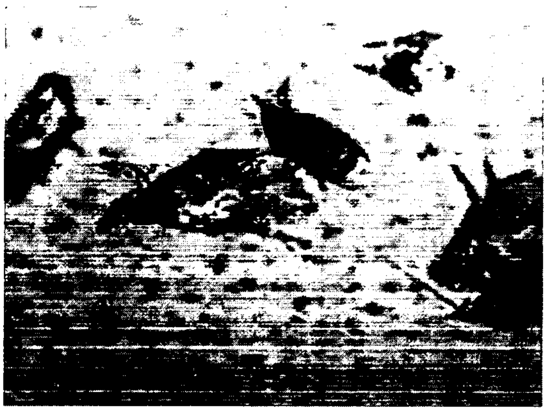

Fig.13.1

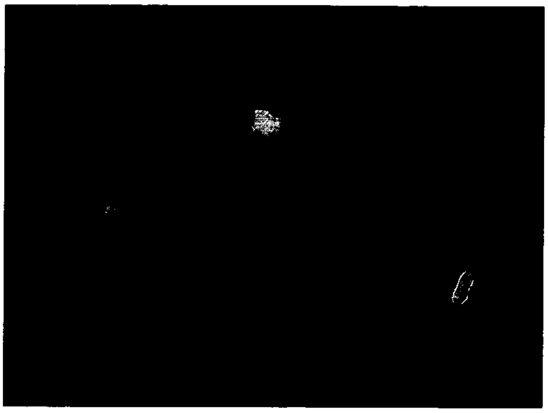

Fig.13.2

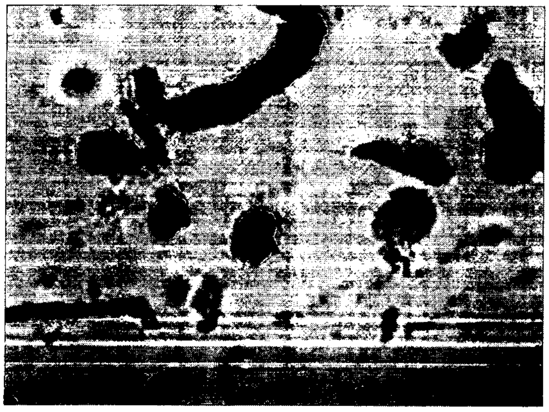

Fig.14.1

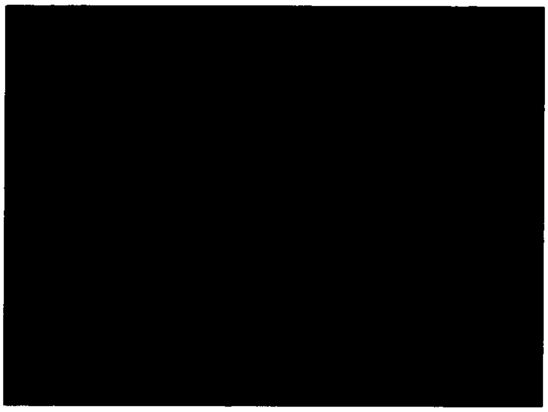

Fig.14.2

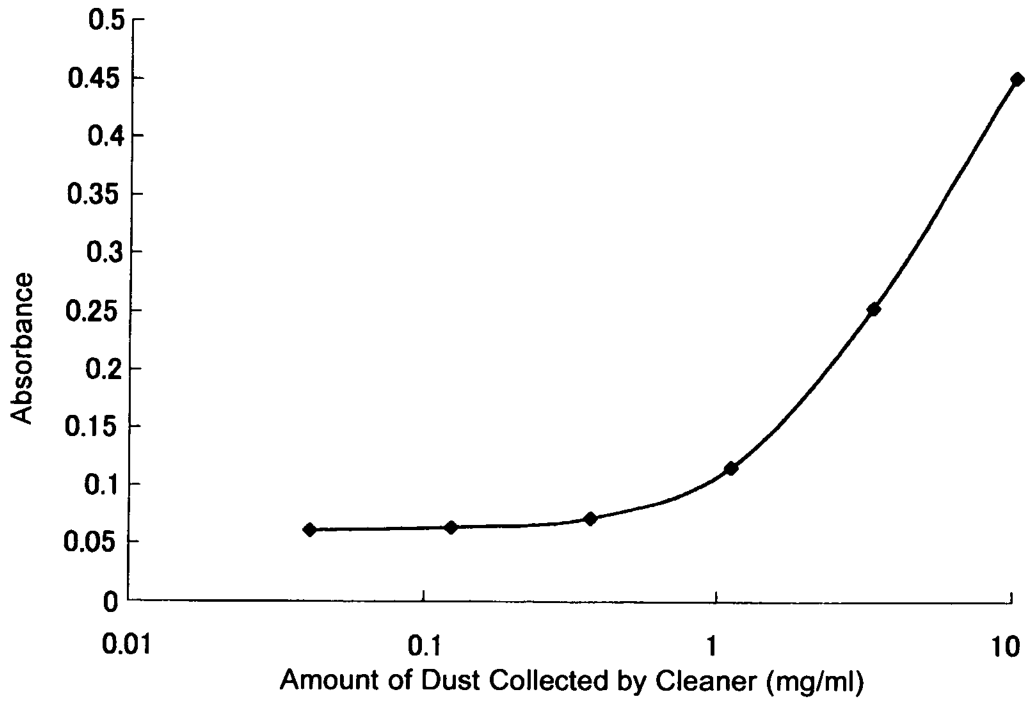
0.5
0.45
0.4
0.35
0.3
0.25
0.2
0.15
0.1
0.05
0
Absorbance
0.01
0.1
1
10
Amount of Dust Collected by Cleaner (mg/ml)

**Fig.15**

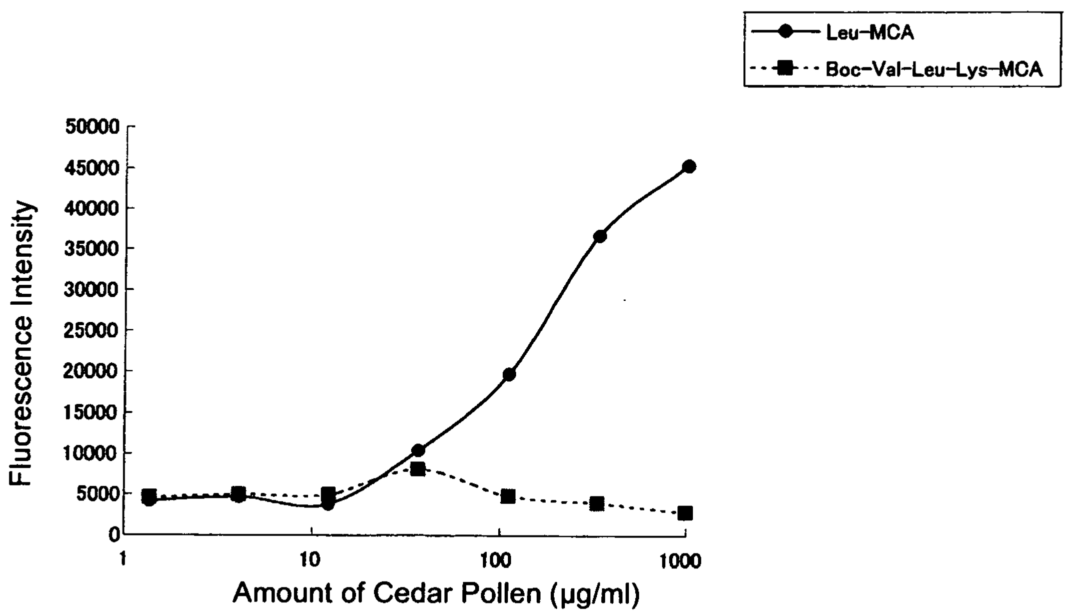
Leu-MCA
Boc-Val-Leu-Lys-MCA
50000
45000
40000
35000
30000
25000
20000
15000
10000
5000
0
Fluorescence Intensity
1
10
100
1000
Amount of Cedar Pollen (μg/ml)

**Fig.16**

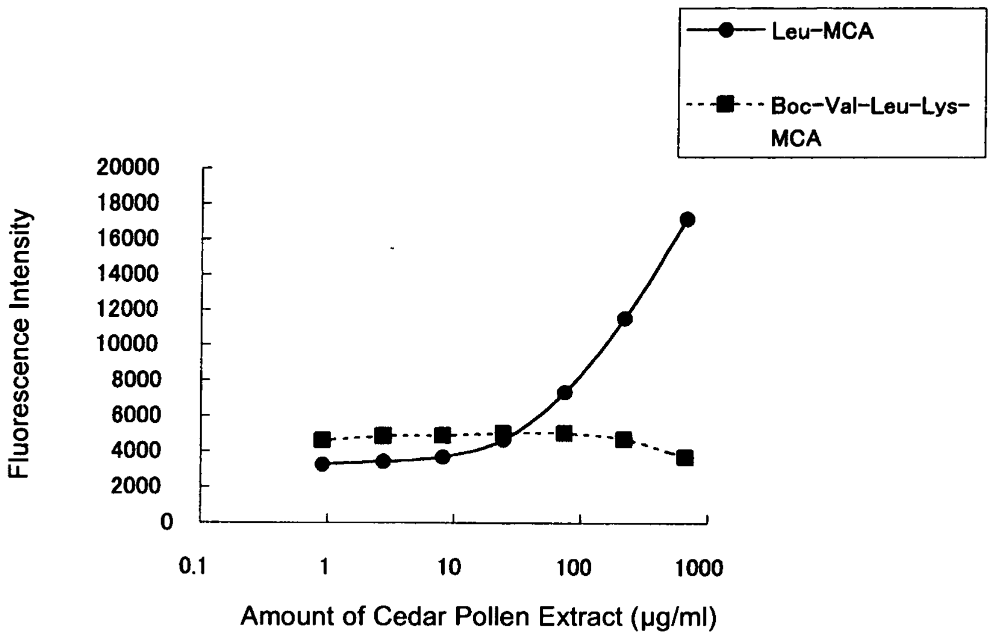
Leu-MCA
Boc-Val-Leu-Lys-MCA
Fluorescence Intensity
20000
18000
16000
14000
12000
10000
8000
6000
4000
2000
0
0.1
1
10
100
1000
Amount of Cedar Pollen Extract (μg/ml)

**Fig.17**

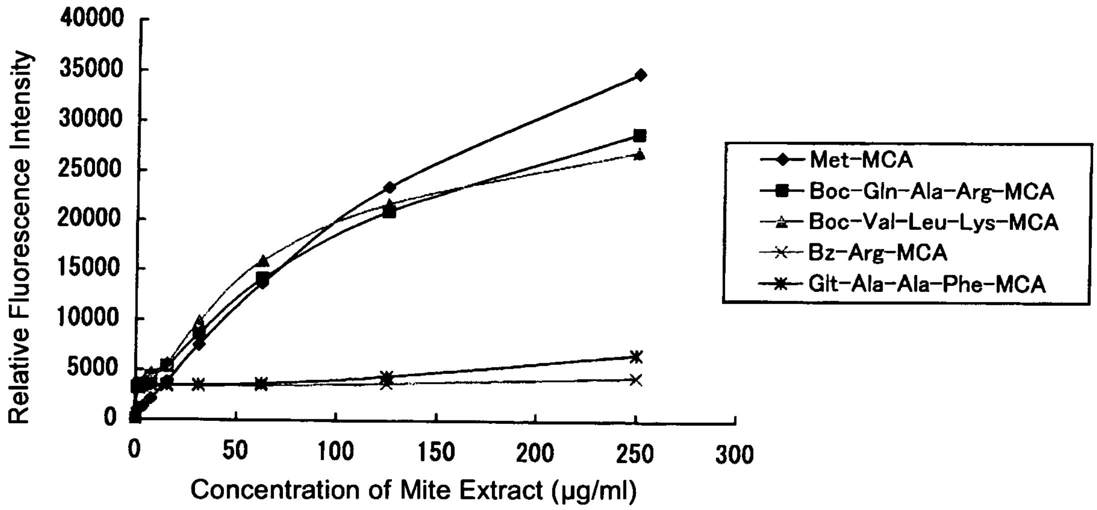
Relative Fluorescence Intensity
40000
35000
30000
25000
20000
15000
10000
5000
0
0
50
100
150
200
250
300
Concentration of Mite Extract (μg/ml)
Met-MCA
Boc-Gln-Ala-Arg-MCA
Boc-Val-Leu-Lys-MCA
Bz-Arg-MCA
Glt-Ala-Ala-Phe-MCA

**Fig.18**

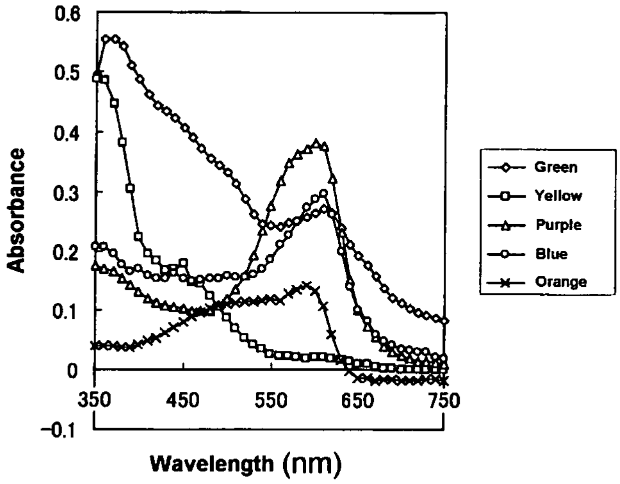


Fig.19

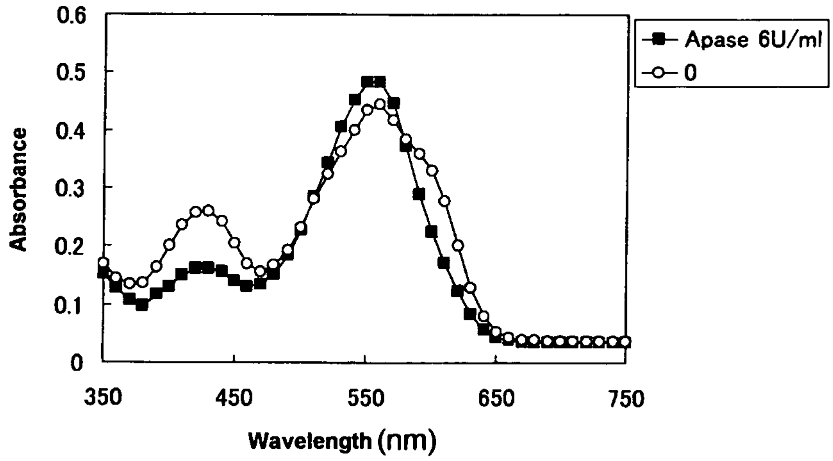


Fig.20

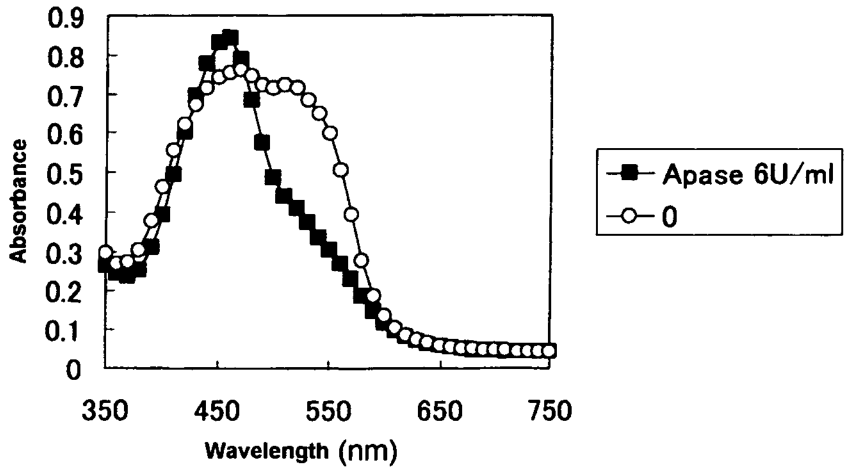


Fig.21

**INTERNATIONAL SEARCH REPORT**

International application No.
PCT/JP2004/018895

A. CLASSIFICATION OF SUBJECT MATTER
Int.Cl$^7$ C12Q1/37, C12M1/34, G01N21/77, 21/78

According to International Patent Classification (IPC) or to both national classification and IPC

B. FIELDS SEARCHED

Minimum documentation searched (classification system followed by classification symbols)
Int.Cl$^7$ C12Q1/37, C12M1/34, G01N21/77, 21/78

Documentation searched other than minimum documentation to the extent that such documents are included in the fields searched

Electronic data base consulted during the international search (name of data base and, where practicable, search terms used)
BIOSIS/MEDLINE/WPIDS(STN)
JSTPLUS(JOIS)

C. DOCUMENTS CONSIDERED TO BE RELEVANT

| Category* | Citation of document, with indication, where appropriate, of the relevant passages | Relevant to claim No. |
|---|---|---|
| X | JP 3-259096 A (Torii Pharmaceutical Co., Ltd.), 19 November, 1991 (19.11.91), (Family: none) | 1-26 |
| X | JP 2003-502641 A (ACARIS HEALTHCARE SOLUTIONS PLC), 21 January, 2003 (21.01.03), & EP 1190245 A1 & WO 00/77516 A1 | 1-26 |
| X | US 5667979 A (LAB LETI SA), 16 September, 1997 (16.09.97), & EP 377229 A1 | 1-26 |
| X | BERRENS L. et al., Estimation of the allergen content of house dust samples by enzymatic assay, Environmental Research, 1991, 56(1), pages 68 to 77 | 1-26 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 February, 2005 (22.02.05) | 15 March, 2005 (15.03.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 9087298 A
- JP 5207892 A
- JP 11014511 A
- JP 2000035428 A
- JP 6034518 A


### Non-patent literature cited in the description


- **NOGUCHI Y ; NAGATA H ; KOGANEI H ; KODERA Y ; HIROTO M ; NISHIMURA H ; INADA Y ; MATSUSHIMA A.** Isolation and characterization of aminopeptidase (Jc-peptidase) from Japanese cedar pollen (Cryptomeriajaponica). *J Agric Food Chem.,* 05 June 2002, vol. 50 (12), 3540-3
- **BAGAROZZI DA JR ; TRAVIS J.** Ragweed pollen proteolytic enzymes: possible roles in allergies and asthma. *Phytochemistry,* February 1998, vol. 47 (4), 593-8
- **MATHESON N ; SCHMIDT J ; TRAVIS J.** Isolation and properties of an angiotensin II-cleaving peptidase from mesquite pollen. *Am J Respir Cell Mol Biol.,* April 1995, vol. 12 (4), 441-8
- **IRANETA SG ; DUSCHAK VG ; RODRIGUEZ SM ; ALONSO A.** Serine proteinases with gelatinolytic activity in an Aspergillus fumigatus allergenic extract. *J Investig Allergol Clin Immunol.,* 2002, vol. 12 (4), 257-62
- **IRANETA SG ; DUSCHAK VG ; RODRIGUEZ SM ; SEOANE MA ; ALBONICO JF ; ALONSO A.** Proteinase and gelatinolytic activities of house dust mite and cockroach extracts. *J Investig Allergol Clin Immunol.,* July 1999, vol. 9 (4), 235-40
- **ANDO T ; INO Y ; HAIDA M ; HONMA R ; MAEDA H ; YAMAKAWA H ; IWAKI M ; OKUDAIRA H.** Isolation of cysteine protease in the crude mite extract, Dermatophagoides farinae. *Int Arch Allergy Appl Immunol.,* 1991, vol. 96 (3), 199-205
- **ANDO T ; HOMMA R ; INO Y ; ITO G ; MIYAHARA A ; YAMAKAWA H ; IWAKI M ; OKUMURA Y ; SUKO M ; HAIDA M et al.** Is a trypsin-like protease of mites a Der f III allergen?. *Arerugi.,* June 1992, vol. 41 (6), 704-7
- **ANDO T ; HOMMA R ; INO Y ; ITO G ; MIYAHARA A ; YANAGIHARA T ; KIMURA H ; IKEDA S ; YAMAKAWA H ; IWAKI M et al.** Trypsin-like protease of mites: purification and characterization of trypsin-like protease from mite faecal extract Dermatophagoides farinae. Relationship between trypsin-like protease and Der f III. *Clin Exp Allergy,* September 1993, vol. 23 (9), 777-84
- **KING C ; SIMPSON RJ ; MORITZ RL ; REED GE ; THOMPSON PJ ; STEWART GA.** The isolation and characterization of a novel collagenolytic serine protease allergen (Der p 9) from the dust mite Dermatophagoides pteronyssinus. *J Allergy Clin Immunol.,* October 1996, vol. 98 (4), 739-47
- **SCHULZ O ; SEWELL HF ; SHAKIB F.** Related Articles, Links A sensitive fluorescent assay for measuring the cysteine protease activity of Der p 1, a major allergen from the dust mite Dermatophagoides pteronyssinus. *Mol Pathol,* August 1998, vol. 51 (4), 222-4
- **YASUEDA H ; MITA H ; AKIYAMA K ; SHIDA T ; ANDO T ; SUGIYAMA S ; YAMAKAWA H.** Allergens from Dermatophagoides mites with chymotryptic activity. *Clin Exp Allergy.,* May 1993, vol. 23 (5), 384-90
- **HEYMANN PW ; CHAPMAN MD ; AALBERSE RC ; FOX JW ; PLATTS-MILLS TA.** Antigenic and structural analysis of group II allergens (Der f II and Der p II) from house dust mites (Dermatophagoides spp. *J Allergy Clin Immunol.,* June 1989, vol. 83 (6), 1055-67
- **STEWART GA ; WARD LD ; SIMPSON RJ ; THOMPSON PJ.** Related Articles, Links The group III allergen from the house dust mite Dermatophagoides pteronyssinus is a trypsin-like enzyme. *Immunology,* January 1992, vol. 75 (1), 29-35